# EUROPÄISCHE PATENTSCHRIFT

(11) **EP 0 791 056 B1**
(45) Veröffentlichungstag und Bekanntmachung des Hinweises auf die Patenterteilung: **30.03.2005**
(21) Anmeldenummer: 95937058.6
(22) Anmeldetag: 09.11.1995
(51) Int. Cl.: C12N 9/16, C07H 19/20, C07H 19/10, A61K 31/70, C12Q 1/44

(54) **LIPIDSPALTENDER ENZYMKOMPLEX**
LIPID-CLEAVAGE ENZYME COMPLEX
COMPLEXE D'ENZYME DE CLIVAGE DE LIPIDES

(30) Priorität: 12.11.1994 DE 4440472; 18.05.1995 DE 19518278
(43) Veröffentlichungstag der Anmeldung: 27.08.1997
(73) Patentinhaber: Heidelberg Pharma GmbH, 69115 Heidelberg (DE)
(72) Erfinder: HERRMANN, Dieter, D-69115 Heidelberg (DE); OPITZ, Hans-Georg, D-69469 Weinheim (DE); ZILCH, Harald, D-68305 Mannheim (DE)
(74) Vertreter: Patentanwälte Zellentin & Partner
(86) Internationale Anmeldenummer: PCT/EP1995/004414
(87) Internationale Veröffentlichungsnummer: WO 1996/015234

(56) Entgegenhaltungen:
- WO-A-92/03462
- WO-A-95/20596
- JOURNAL OF BIOLOGICAL CHEMISTRY, Bd. 265, Nr. 11, 1990, MD US, Seiten 6112-6117, XP000371928 HOSTETLER K.Y. ET AL.: "Synthesis and antiretroviral activity of phospholipid analogs of azidothymidine and other antiviral nucleosides"
- JOURNAL OF MEDICINAL CHEMISTRY, Bd. 34, 1991, WASHINGTON US, Seiten 1408-1414, XP002000878 PIANTADOSI C. ET AL. : "Synthesis and evaluation of novel ether lipid nucleoside conjugates for anti-HIV-1 activity"
- GRIENGL H. ET AL: 'Phosphonoformate and phosphonacetate derivatives of 5-substituted 2'-deoxyuridines: Synthesis and antiviral activity' J.MED.CHEM Bd. 31, 1988, US, Seiten 1831 - 1839
- IYER R.P. ET AL: 'Synthesis, hydrolytic behavior, and anti-HIV activity of selected acyloxyalkayl esters o f trisodium phosphonoformate' JOURNAL OF PHARMACEUTICAL SCIENCES Bd. 83, Nr. 9, 09 September 1994, US, Seiten 1269 - 1273

## Beschreibung

Der lipidspaltende Enzymkomplex (Lipid-Cleavage-Enzym; LCE), Gegenstand der vorliegenden Erfindung, ist ein bisher nicht beschriebenes, membranständiges Enzym, das z. B. aus Zellmembranfraktionen von humanen peripheren Blutleukozyten oder humanen Makrophagen isoliert werden kann, und die Konjugate von pharmakologisch aktiven Substanzen, die an ein lipidartiges Trägermolekül gebunden sind, unter Freisetzung der pharmakologisch aktiven Substanz oder dessen Monophosphat spalten. Außerdem betrifft die Erfindung die Verwendung dieser Konjugate, die als Substrate dieses Enzymkomplexes dienen, zur Herstellung von Arzneimitteln, wobei die Arzneimittel diese Konjugate als pharmazeutischen Wirkstoff enthalten. Die Arzneimittel eignen sich zur gezielten Freisetzung und Anreicherung von pharmakologisch aktiven Substanzen in entsprechenden Zielzellen. Gegenstand der Erfindung sind ferner In-vitro-Untersuchungssysteme, die diesen Enzymkomplex enthalten, zur Auffindung von weiteren Substraten dieses Enzymkomplexes. Unter LCE wird sowohl der Enzymkomplex, isoliertes Enzym und mögliche Isoenzyme verstanden.

Die Therapie bösartiger Neoplasien (z. B. Karzinome, Sarkome, Hämoblastosen, hämatologische Neoplasien), entzündlicher Erkrankungen oder Autoimmunerkrankungen sowie von durch Viren oder Retroviren hervorgerufenen Erkrankungen, wie beispielsweise von AIDS, ARC (AIDS related complex), Cytomegalie, Herpes oder Hepatitis, ist neben der unzureichenden Wirksamkeit der eingesetzten therapeutischen Wirkstoffe häufig mit deren extremen Nebenwirkungen verbunden. Dieser Effekt ist mit der zu geringen In-vivo-Selektivität bzw. der eingeschränkten therapeutischen Breite der eingesetzten pharmakologisch aktiven Substanzen zu erklären. Die günstigen pharmakologischen In-vitro-Eigenschaften der pharmakologisch aktiven Substanzen sind oft nicht auf die Invivo-Verhältnisse übertragbar.

Seit Jahren versucht man deshalb durch Modifzierung der chemischen Struktur von pharmakologisch aktiven Substanzen neue Substanzen zur Verfügung zu stellen, die verbesserte Eigenschaften hinsichtlich der therapeutischen Breite aufweisen. Ferner werden oft neue pharmazeutische Darreichungsformen mit dem Ziel entwickelt, die aktiven Substanzen gezielt an ihren Wirkungsort zu transportieren, an dem sie ihre therapeutische Wirkung entfalten sollen. Dabei soll insbesondere die unerwünschte Wechselwirkung mit gesunden Zellen vermieden werden. Im Falle von Tumorzellen, die entsprechende Oberflächenantigene besitzen, wurden beispielsweise Antikörper hergestellt, die diese speziellen Oberflächenantigene erkennen und somit gezielt an die Krebszelle binden. Die Antikörper sind mit geeigneten Toxinen derart modifiziert, daß nach erfolgter Bindung an die Krebszelle das Toxin freigesetzt und die Krebszelle mortalisiert wird. Eine andere Alternative zur Verbesserung der therpeutischen Breite besteht darin, durch geringfügige Modifizierung der pharmakologisch aktiven Substanz, beispielsweise durch Herstellung von Säure- oder Basenadditionssalzen oder durch Herstellung von einfachen Estern [beispielsweise Fettsäureester; J. Pharm. Sci. 79, 531 (1990)], die physikalischen Eigenschaften der zugrundeliegenden aktiven Substanz derart zu verändern, daß die Löslichkeit oder Verträglichkeit der aktiven Substanz verbessert wird. Diese geringfügig chemisch modifizierten Verbindungen werden oft auch als sogenannte "Prodrugs" bezeichnet, da sie beim Kontakt mit Körperflüssigkeiten oder in der Leber (first pass-Metabolismus) nahezu unmittelbar in das eigentliche therapeutisch aktive Agens umgewandelt werden.

Das der vorliegenden Erfindung zugrundeliegende technische Problem bestand darin, ein neues Target aufzufinden, das möglichst spezifisch auf oder in Zellen vorkommt, die ein Zielobjekt für die Verabreichung von pharmakologisch aktiven Substanzen darstellen. Das Target sollte mit entsprechenden pharmazeutischen Wirkstoffen in Wechselwirkung treten, so daß die Wirkstoffe möglichst spezifisch zu diesen Zielzellen transportiert, von diesen erkannt, gebunden und aufgenommen werden können. Der pharmazeutische Wirkstoff sollte dabei im wesentlichen aus zwei Bestandteilen aufgebaut sein, wobei der erste Bestandteil für die Erkennung und Wechselwirkung mit dem Target verantwortlich ist (ligandspezifischer Teil) und der zweite Bestandteil die eigentliche aktive Substanz (wirkstoffspezifischer Teil) darstellt, der erst dann seine Wirkung entfaltet, wenn die spezifische Bindung an das Targetmolekül und die intrazelluläre Abspaltung des eigentlich aktiven Agens oder dessen Monophosphates erfolgt ist. Dadurch soll die unerwünschte Freisetzung der pharmakologisch aktiven Substanz in den Körperflüssigkeiten vermieden werden, so daß gesunde Zellen nicht durch das pharmakologisch aktive Agens negativ beeinflußt und unerwünschte Nebenwirkungen weitgehend vermieden werden. Der für diesen Zweck für die Herstellung der Arzneiform verwendete pharmazeutische Wirkstoff sollte hierbei einerseits als Ligand für das Target dienen und andererseits das eigentliche aktive pharmakologische Agens enthalten, wobei dieses insbesondere auch auf Basis von bereits bekannten pharmazeutischen Wirkstrukturen beruhen kann, deren therapeutische Breite auf diese Weise signifikant verbessert werden soll.

Überraschenderweise wurde nun gefunden, daß sich als Target LCE eignet, wobei LCE vorwiegend auf oder in malignen, aktivierten oder virusinfizierten Zellen, insbesondere auf humanen peripheren Blutleukozyten, Makrophagen, Nieren-, Nebennieren- oder Ovarialzellen, Zellen des lymphatischen Systems oder der lymphoiden Organe oder Zellen des Gehirns lokalisiert ist. Dieser Enzymkomplex und seine Analoga werden im folgenden auch kurz als LCE bezeichnet. LCE zeigt überraschenderweise keine über alle Organe verteilte gleichmäßige statistische Verteilung, sondern ist vorwiegend in Membranen von bestimmten Zellen zu beobachten, die als Zielobjekt für die Verabreichung von pharmakologisch aktiven Substanzen in Frage kommen. In Herz-, Knochenmarkund Leberzellen konnte eine vergleichsweise nur sehr geringe Enzymaktivität festgestellt werden. Die Homogenat- bzw. Membranfraktionen der zur Isolierung des LCE verwendenten Zellen, Organe oder Gewebe zeigen verschiedene spezifische Aktivitäten oder Affinitäten des LCE, wobei in aktivierten Zellen die spezifische Aktivität oder die Affinität stark erhöht sind im Vergleich zu nicht-aktivierten Zellen. Dies läßt sich für humane periphere Blutleukozyten-, Lymphozyten und Granulozyten sowie für humane und nicht-humane bzw. murine mononukleäre Zellen, wie z. B. Monozyten/Makrophagen belegen.

LCE zeichnet sich überraschenderweise dadurch aus, daß es als Substrat lipidähnliche Verbindungen zwischen dem Lipidgrundgerüst und der Verbrückungsstruktur einer kovalent an diese Brücke gebundenen, physiologisch aktiven Substanz spaltet. Derartige Substrate können durch die allgemeine Formel I

L - B - D (I)

umschrieben werden, wobei L für einen Lipidrest, B eine Brücke und D für eine pharmakologisch aktive Substanz steht bzw. B-D ein Wirkstoffphosphonat darstellt. Überraschenderweise besitzen die pharmazeutischen Wirkstoffe der Formel I im Vergleich zu den pharmakologisch aktiven freien bzw. unmodifizierten Substanzen D oder -B-D eine größere therapeutische Breite. Sie verbessern darüberhinaus oft deren Verweilzeit im Körper, die Bioverfügbarkeit oder die oft als kritischer Faktor bekannte Membrangängigkeit (z.B. Blut-Hirn-Schranke, Zellmembranen, etc.) der pharmakologisch aktiven Substanzen. Substrate der Formel I dienen somit als Trägersystem (Carrier) für die pharmakologisch aktive Substanz. Die Konjugate der Formel I können hinsichtlich ihrer Funktion als intrazelluläres Drug-Storage-, Drug-Targeting- und Drug-Delivery-System bezeichnet werden. Sie bewirken, daß die pharmakologisch aktive Substanz oder deren Prodrugform nach oraler Applikation intrazellulär freigesetzt wird, wobei diese Freisetzung in vorteilhafter Weise nicht unspezifisch in allen Zellen, Organen oder Geweben des Körpers stattfindet, sondern gezielt in solchen Zellen, die das LCE in der Zellmembran oder auch teilweise intrazellulär enthalten. Besonders überraschend ist jedoch, daß die Spaltung nicht bereits schon während des Transportes des Substrates durch die Körperflüssigkeiten, wie z.B. Blut, Serum oder Lymphflüssigkeit oder durch die Leber, erfolgt, sondern erst an oder in den entsprechenden Zielzellen. Auf diese Weise wird das unerwünschte Ausscheiden des Spaltproduktes durch die Niere oder Spaltung des Substrates in der Leber vermieden, so daß der weitaus größte Teil des Wirkstoffes an die jeweiligen Zielzellen transportiert wird. Derartige Zellen sind, wie oben bereits erwähnt, insbesondere pathophysiologisch oder physiologisch aktivierte Zellen, die als Zielobjekt für die Verabreichung von pharmakologisch aktiven Substanzen in Frage kommen, wie beispielsweise Blutleukozyten, Lymphozyten, Makrophagen und andere Zellpopulationen des immunologisch lymphatischen Systems. Insbesondere handelt es sich hierbei um aktivierte Zellen (z.B. Makrophagen, Granulozyten, Lymphozyten, Leukozyten, Thrombozyten, Monozyten etc.), die beim jeweiligen Krankheitsgeschehen eine pathologische, physiologische, pathophysiologische oder symptomatische Rolle spielen.

Bei LCE handelt es sich um einen bisher noch nicht beschriebenen Enzymkomplex. Er zeichnet sich insbesondere dadurch aus, daß er als Substrat die Verbindung (3'-Desoxy-3'-azidothymidin)-5'-phosphorsäure-(3-dodecyl-mercapto-2-decyloxy)-propylester (nach- folgend kurz als AZT-DMDOPE bezeichnet) zu 3'-Desoxy-3'-azidothymidin-monophosphat und (3-dodecylmercapto-2-decyloxy)-propanol (DMDOP) spaltet. Ein weiteres bevorzugtes Substrat von LCE ist die Verbindung (3'-Desoxy-3'-fluorthymidin)-5'-phosphorsäure-(3-dodecylmercapto-2-decyloxy)-propylester (FLT-DMDOPE), das zu 3'-Desoxy-3'-fluorthymidin-5'monophosphat und DMDOP gespalten wird. Alternativ wird auch (5-Fluoruridin)-5'-phosphorsäure-(3-dodecylmercapto-2-decyloxy)-propylester (5-FU-DMDOPE) zu (5-Fluoruridin)-5'-monophosphat (5-FU-MP) und DMDOP gespalten.

Die hergestellten LCE-haltigen Präparationen sind frei von Phospholipase C.
Dies konnte sowohl durch verschiedene Kationenabhängigkeit der LCE-Aktivität. wie durch Phospholipase C-spezifische Inhibitoren, die LCE nicht inhibieren, gezeigt werden.

Zur Bestimmung des Umsatzes von AZT-DMDOPE durch Zellhomogenate, Membran- bzw. Zytosolfraktionen, vorzugsweise von verschiedenen humanen Zelltypen, wurde ein Enzym-Assay etabliert (Beispiel 6).

Das Testprinzip beruht auf einer Spaltung der Muttersubstanz durch das LCE in AZT-MP und den korrespondierenden Thioetherlipidteil. [¹⁴C]-AZT-DMDOPE und nichtradioaktiv markiertes AZT-DMDOPE wurden hierfür eingesetzt. Der Thioetherlipidmetabolit DMDOP wurde aus den Ansätzen extrahiert (Beispiel 7) und die Menge an radioaktiv markierter Substanz im Flüssigkeits-Szintillations-Analysator gemessen. Da eine definierte Menge an [¹⁴C]-AZT-DMDOPE im Enzym-Assay eingesetzt wurde konnte der Umsatz der enzymatischen Spaltung bestimmt werden.

Das Enzym wird vorzugsweise aus humanen peripheren Blutleukozyten, die zuvor durch PHA oder andere Stimulantien (z.B. Cytokine, etc.) aktiviert wurden, oder aus murinen Nierenzellen isoliert. Aus vorläufigen Untersuchungen ergibt sich ein Molekulargewicht von etwa 120.000 - 160.000, bestimmt nach der SDS-PAGE-Methode. Zellen, die dieses Enzym enthalten, können auf einfache Weise dadurch identifiziert werden, indem sie unter geeigneten Testbedingungen mit einer Lösung von AZT-DMDOPE oder FLT-DMDOPE versetzt werden und die Spaltprodukte AZT-monophosphat bzw. FLT-monophosphat oder DMDOP nachgewiesen werden, beispielsweise durch dünnschichtchromatographische Methoden oder im Fall von radioaktiv markierten Proben durch szintillatographische Verfahren (siehe Beispiele 4 - 7). Im Gegensatz zu den biochemisch verwandten Phospholipasen C oder -D wird das LCE nicht durch bekannte Inhibitoren von Phospholipase C oder -D gehemmt oder durch Aktivatoren von Phospholipase C oder -D aktiviert. Im Gegensatz zu Phospholipase C wird das Enzym durch die Substanz D 609 (Tricyclodecan-9-yl-xanthogenat; C₁₁H₁₅OS₂ K) aktiviert, während D 609 Phospholipase C inhibiert.

Intrazelluläres AZT zeigt in seiner nichtphosphorylierten Form keine inhibitorische Wirkung auf die virale reverse Transkriptase (RT) (Nakashima et al., 1986, Antimicrob. Agents Chemother. 30, 933-937; Mitsuya et al., 1985, Proc. Natl. Acad.Sci. USA 82, 7096-7100). Durch die intrazellulären Enzyme Thymidin-Kinase, Thymidilat-Kinase und Pyrimidin-Nukleosid-Diphosphat-Kinase (Yarchoan et al., 1989, N. Engl. J. Med. 321, 726-738; Toyoshima et al., 1991, Anal. Biochem. 196, 302-307) wird das Strukturanalogon von Thymidin durch sukzessive Phosphorylierung über AZT-MP und AZT-DP in das therapeutisch wirksame, RT inhibierende AZT-TP überführt. AZT-DMDOPE, als Thioetherlipid-AZT-Konjugat, könnte durch intrazelluläre enzymatische Spaltung in AZT oder direkt in die bereits phosphorylierte Form AZT-MP überführt werden. In Beispiel 8 werden die intrazellulären Konzentrationen an phosphoryliertem und nichtphosphoryliertem AZT nach Inkubation mit äquipotenten Konzentrationen an AZT-DMDOPE und AZT bestimmt.

Der Enzymkomplex und seine Analoga zeigen ferner in verschiedenen Spezies (z. B. Mensch, Maus, Ratte, Hund, Affe) keine gleichmäßige statistische Organverteilung, sondern sind nur in den Membranen von bestimmten Zellen, Organen oder Geweben vorzufinden, die als Targetzellen für spaltbare Konjugate der Formel I dienen. Die natürlichen Substrate dieser Enzyme sind bislang noch unbekannt. In zytosolischen Fraktionen liegt die LCE-Aktivität immer unter oder gerade an der Nachweisgrenze. Sehr geringe Aktivität ist z.B. auch in Knochenmarkzellen festzustellen, was auf eine sehr geringe oder sogar gänzlich fehlende Knochenmarktoxizität der als Substrate des LCEs eingesetzten pharmazeutischen Wirkstoffe der Formel I schließen läßt.

Die LCE-Aktivität zeigt eine lineare Protein- und Zeitabhängigkeit, eine spezifische Abhängigkeit von Metallkationen (Inhibierung durch Ca²⁺, Zn²⁺ und Mn²⁺) und eine klassische Michaelis-Menten-Kinetik (Substratabhängigkeit) (Beispiel 9). Neben der höheren Aktivität des Enzymkomplexes in aktivierten Zellen zeigt sich unter diesen Bedingungen auch eine höhere Affinität des LCE zum Substrat.

Das isolierte oder aus Membranfraktionen stark angereicherte LCE läßt sich auch zum Screening auf neue, potentiell spaltbare Substrate oder auch natürlicherweise vorkommende Substrate einsetzen. Die so gefundenen Substrate können dann weitergehend untersucht werden hinsichtlich ihrer essentiellen Strukturmerkmale, die für die Erkennung des Substrates und die Bindung an LCE erforderlich sind. Derartige identifizierte Struktureigenschaften können dann verwendet werden, um chemisch modifizierte Substrate herzustellen, die diese essentiellen Merkmale enthalten, sowie darüber hinaus geeignete funktionelle Gruppen, die sich zur Kopplung mit pharmakologisch aktiven Substanzen eignen.

Auch ein Screening auf Inhibitoren oder Aktivatoren des Lipid-Cleavage-Enzym ist somit möglich.

Diagnostisch kann das isolierte oder stark angereicherte Enzym eingesetzt werden, wenn beispielsweise gesteigerte oder reduzierte Lipid-Cleavage-Enzym-Aktivitäten zu pathologischen Veränderungen in vitro oder in vivo führen, oder mit diesen pathologischen Veränderungen entsprechende Erkrankungen oder Krankheitssymptome einhergehen.

Ebenso kann das LCE zur Herstellung von diagnostischen Mitteln eingesetzt werden, die dazu verwendet werden, um bei der Verabreichung der pharmazeutischen Wirkstoffe an Patienten die Spaltung dieser Substrate zu prüfen, was zur spezifischen und individuellen Anpassung der Therapiemodalitäten bei diesen Patienten führt (Drugmonitoring).

Das LCE zeichnet sich insbesonders dadurch aus, daß es Verbindungen des allgemeinen Typs I 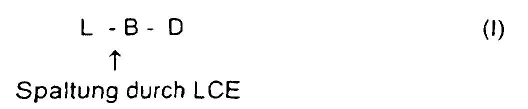 spalten kann, in dem L einen Lipidteil darstellt, B eine Brücke kennzeichnet und D für eine pharmakologische aktive Substanz steht bzw. B-D ein Wirkstoffphosphonat bedeutet. Die sehr spezifische Spaltung erfolgt zwischen Lipidteil und Phosphatrest. Eine unspezifische Spaltung der Verbindungen der Formel I an anderen funktionellen Gruppen im Molekül wird nicht beobachtet.

Die Brücke B wird durch die Formel III umschrieben,

-O-[PZ (OH)A]ₙ- (III)

in der n = 1, 2 oder 3 sein kann, aber bevorzugt gleich 1 oder 2 ist und insbesondere 1, Z gleich O oder S, und A entweder O, S oder ein Valenzstrich, bevorzugt O ist.

Der Begriff "pharmakologisch aktive Substanz" (Bezeichnung D bzw. B-D bei Phosphonaten in Formel I) steht in dieser Anmeldung für einen Wirkstoff im arzneimittelrechtlichen Sinne. Dieser Wirkstoff kann ein Wirkstoff eines bereits eingeführten, von Arzneimittelbehörden zugelassenen Pharmazeutikums oder ein sich in der arzneimittelrechtlichen Zulassung befindlicher Wirkstoff sein. Die Definition "pharmakologisch aktive Substanz" umfaßt auch solche Derivate von Wirkstoffen, die durch Einführung einer oder mehrerer funktioneller Gruppen (beispielsweise solche Gruppen, die eine Kupplung von D mit dem Lipid-Carrier-Teil L ermöglichen, wie z.B. Hydroxy- oder Aminogruppen) chemisch modifiziert werden können. Die Definition umfaßt ferner die aus dem Wirkstoff D sich bildenden Prodrugformen, die ebenfalls physiologisch aktiv sind. Insbesondere kommen solche pharmakologisch aktive Substanzen D in Frage, deren klinische Entwicklung aufgrund unerwünschter Nebenwirkungen eingestellt oder nicht begonnen wurde, bzw. die über ein sehr enges Dosis-Wirkungs-Spektrum verfügen, so daß die Verabreichung der therapeutisch erforderlichen Menge nur mit hohen Risiken oder praktisch überhaupt nicht beherrschbar war.

Überraschenderweise wurde gefunden, daß die therapeutische Breite einer pharmakologisch aktiven Substanz D bzw. B-D im Falle von Wirkstoffphosphonaten signifikant verbessert wird, wenn die Substanz an ein lipidartiges Trägermolekül gekoppelt wird. Das so hergestellte Konjugat dient als neuer Wirkstoff für die Herstellung von pharmazeutischen Darreichungsformen. Insgesamt resultiert aus der Kopplung eine verstärkte Wirkung der pharmazeutisch aktiven Substanz D bzw. B-D in vivo, da durch das entstehende Drug-Delivery-Transport-System eine Lokalisierung der pharmakologisch aktiven Substanz in Zielzellen erfolgt und dadurch die pharmakologisch aktive Substanz hinsichtlich ihrer Effizienz gesteigert wird. Dies bedeutet, daß einerseits die Menge der zu verabreichenden pharmakologisch aktiven Substanz reduziert werden kann, oder andererseits unter Beibehaltung der gleichen effektiven Menge eine verstärkte pharmakologische Wirkung erzielt wird.

Die den pharmakologisch aktiven Substanzen D bzw. B-D zugrundeliegende chemische Struktur kann ferner derart modifiziert werden, daß die Substanzen hinsichtlich ihrer physikalischen oder chemischen Eigenschaften verändert sind und beispielsweise eine höhere oder geringere Lipophilie aufweisen, jedoch hinsichtlich ihrer therapeutischen Wirkung im wesentlichen die gleichen Eigenschaften besitzen wie die unmodifizierte Substanz D bzw. B-D. Insbesondere ist es vorteilhaft, wenn die Substanz D durch Einführung von funktionellen Gruppen chemisch derart modifiziert wird, daß sie über eine geeignete Brücke an den Lipidteil L gekoppelt werden kann. Dies geschieht beispielsweise durch Einführung von Hydroxygruppen, die über die Phosphatgruppe B an das Lipid gekoppelt werden.

Die pharmakologisch aktive Substanz D bzw. B-D stellt eine chemische oder auf biologischer Basis (Antikörper, Peptid, Protein, Hormon, Toxin etc.; INDEX NOMINUM, International Drug Directory, Medpharm) aufgebaute Substanz mit biologischer Wirkung dar, sowie deren durch Einführung einer funktionellen Gruppe (z.B. einer Hydroxygruppe) chemisch modifizierten Derivate. Eine Voraussetzung ist, daß die pharmakologisch aktive Substanz oder ihre Prodrugform über die Spaltung des Liponukleotids durch das Lipid-Cleavage-Enzym eine "Aktivierung" durch dieses körpereigene Enzym erfahren. Bevorzugt sind dabei pharmakologisch aktive Substanzen, die nach Spaltung durch das LCE als pharmakologisch aktives Substanzmonophosphat in vivo als Zwischenprodukt fungieren und durch zelluläre Enzyme weiter aufphosphoryliert werden, wie z.B. Nukleosid-Monophosphat zum Nukleosid-Triphosphat, oder zur freien pharmakologisch aktiven Substanz gespalten werden (siehe Beispiel 8).

Im Sinne der Erfindung kommen insbesondere alle in vitro wirksamen, jedoch in vivo im therapeutischen Bereich toxischen pharmakologisch aktive Substanzen in Frage, d.h. alle Substanzen mit kleiner therapeutischer Breite, die über eine chemisch funktionelle Gruppe zur Knüpfung der kovalenten Bindung zum Phosphat verfügen. Außerdem können auch solche Substanzen verwendet werden, die in ihrer pharmakologisch aktiven Form zunächst keine funktionelle Gruppe enthalten, diese sich jedoch durch chemische Modifikation einführen läßt, ohne daß ein Verlust der Wirkung der Substanz eintritt.

Bevorzugt werden solche pharmakologisch aktive Substanzen zur Konjugation mit einem Lipidrest L verwendet, die normalerweise nach Phosphorylierung ihre aktive Form erreichen (wie z.B. im Fall von Nukleosiden) bzw. Wirkstoffphosphonate. Aus dem Konjugat wird dann das pharmakologisch aktive Substanzphosphat durch enzymatische Hydrolyse des Konjugats freigesetzt. Die Freisetzung der phosphorylierten Substanz ist insbesondere deshalb von Bedeutung, da dieser Prozeß auch in solchen Zellen stattfinden kann, die nicht über die normalerweise zur Aufphosphorylierung der reinen pharmakologisch aktiven Substanz nötigen Enzyme (Kinasen) verfügen. Die konjugierte und via LCE intrazellulär oder in der Zellmembran freigesetzte pharmakologisch aktive Substanz kann beispielsweise eine zytostatische, zytotoxische, antitumorale, antivirale, antiretrovirale, immunsupprimierende oder immunstimulierende Wirkung aufweisen.

Als pharmakologisch aktive Substanzen D bzw. B-D kommen alle Substanzen in Frage mit kurzer Halbwertszeit, insbesondere auch Verbindungen mit unterschiedlicher Organ-, Gewebe-, oder Zellhalbwertszeiten; mit schlechter Bioverfügbarkeit, d.h. mit schlechter Resorption, hoher Leberspaltung oder schneller Elimination; mit schlechter Membranpenetration (z.B. Zellmembran, Blut-Hirnschranke); mit Knochenmarktoxizität oder anderen limitierenden Organtoxizitäten (z.B. Cardio-, Leber-, Nephro-, Neurotoxizität etc.), deren Wirkkonzentration in vivo zu gering ist. Außerdem sind solche Substanzen geeignet, die gezielt mit dem Zellkern der Zielzellen in Wechselwirkung treten und auf der Ebene der DNA oder RNA in das molekulare Geschehen eingreifen, wie z.B. Antisense-Oligonukleotide, DNA-Fragmente, und die für die Gentherapie verwendet werden können.

Verbindungen der Formel I und deren Herstellung sind in den Anmeldungen WO 92/03462, WO 93/16092, WO 93/16091, WO 94/03465, PCT/EP94/02123, DE 4402492, DE 4418690, sowie beispielsweise in WO 91/19726; EP 0 350 287; US 5,223,263; US 5,194,654; US 4,921,951; US 4,622,392; US 4,291,024; US 4,283,394 beschrieben. Im Falle von antiviral wirksamen Nucleosiden werden in EP 0 350 287 und US 5,223,263 Lipid-Derivate (Diacylglycerol-Nucleoside) und deren Verwendung in liposomaler Form beschrieben. Bevorzugt in Form von Liposomen soll eine Aufnahme der Substanz durch Zellen des retikuloendothelialen Systems (RES), z. B. Makrophagen und Monozyten möglich sein.

Durch entsprechende Vergleichsversuche konnte gezeigt werden, daß die therapeutischen Effekte der aus EP 0 595 133 bekannten Diacylglycerol-Konjugate in vivo den Thioether-oder Etherlipid-Konjugaten aus WO92/03462 unterlegen sind. Dies ist auf eine unspezifische, nicht nur am Wirkort stattfindende Hydrolyse der Fettsäureester zurückzuführen. Die nicht-hydrolysierbaren Thioether- und Etherreste zeigen demgegenüber entscheidende Vorteile, da erst durch das spezielle Enzym (LCE) in den Membranen des Zielgewebes bzw. intrazellulär eine Substanz mit biologischer Wirkung oder entsprechende Zwischenprodukte freigesetzt werden.

Die Konjugate der Formel I (L-B-D) zeigen entscheidende Vorteile im Vergleich zur nicht konjugierten pharmakologisch aktiven Substanz D bzw. B-D: Der spezifische, kovalent an die pharmakologisch aktive Substanz gebundene Carrier (L-B- bzw. L) verbessert die Bioverfügbarkeit von schlecht resorbierten pharmakologisch aktiven Substanzen, die Verträglichkeit von potentiell toxischen Wirkmolekülen, die Verweilzeit von schnell eliminierten oder metabolisierten Arzneimitteln und die Membranpenetration von schlecht membrangängigen Verbindungen (z.B. Blut-Hirn, Zellen etc.). Die enzymatische Spaltung in vivo in Carrier und pharmakologisch aktive Substanz D (bzw. Substanz-Derivat) bzw. in Carrier und pharmakologisch aktives Substanzphosphat (D-Monophosphat) oder Phosphonat B-D erfolgt in der Regel nicht im Serum sondern erst intrazellulär. Außerdem verbessert der Carrierteil mit seiner lecithinartigen Struktur, die für den beanspruchten Effekt essentiell ist, die Penetration oder Membrangängigkeit der pharmakologisch aktiven Substanz und zeigt in vielen Fällen einen Depoteffekt. Darüberhinaus ist die gastrointestinale Verträglichkeit der Lipidkonjugate L-B-D vielfach besser als die der reinen pharmakologisch aktiven Substanzen D. Auch bei der Resorption zeigt das Lipid-Konjugat eine bessere Penetration durch Membranstrukturen und somit eine bessere Überwindung der Resorptionsbarrieren. Entsprechendes gilt für die Penetration z.B. der Blut-Hirn-Schranke durch erleichterte Diffusion oder eventuell aktiven Transport.

Sowohl die Gesamtkörper-, Zell- als auch die Organ-Halbwertszeiten der pharmakologisch aktiven Substanz werden durch Konjugation aufgrund der längeren Verweilzeit des Konjugats im Organismus erheblich verlängert. Aufgrund der fehlenden LCE-Aktivität zur Spaltung im Serum und in einigen Organen ist nahezu keine bzw. nur eine sehr geringere Knochenmark- und Organtoxizität zu beobachten. Insbesondere ist vorteilhaft, daß die Konjugate der Formel I in verschiedenen Zielorganen, Geweben oder Zellen spezifisch angereichert werden.

Die Verbindungen der Formel I können als Wirkstoffe zur Herstellung von Arzneimitteln verwendet werden, die für alle Erkrankungen eingesetzt werden, bei denen hohe pharmakologisch aktive Substanzspiegel in Zellen, Organen oder Geweben erforderlich oder hilfreich sind. Eine wesentliche Voraussetzung für dieses als "Drug-Storage-Delivery-Targeting" zu bezeichnende Transportsystem ist, daß die im Sinne der beabsichtigten Therapie anzusprechenden Zellen das Lipid-Cleavage-Enzym in den inneren oder äußeren Zellmembranen aufweisen, so daß in einem ersten Schritt der Wirkstoff an das LCE bindet und anschließend über die Zellmembran hinweg in das Innere der Zelle transportiert wird, wobei die Spaltung des Wirkstoffes zu der physiologisch aktiven Substanz entweder im wesentlichen gleichzeitig mit dem Transport über die Zellmembran oder auch später teilweise innerhalb der Zelle erfolgt. Die intrazelluläre Spaltung kommt insbesondere in solchen Fällen vor, bei denen das LCE auch innerhalb der Zelle lokalisiert ist. Im Sinne der Erfindung kann die Spaltung des Wirkstoffes verbunden mit der intrazellulärer Freisetzung der pharmakologisch aktiven Substanz auch derart erfolgen, daß entweder die physiologisch oder pharmakologisch aktive Substanz dabei direkt entsteht oder eine dieser Substanz entsprechende Vorstufe (Prodrugform). Geeignete Zielzellen sind beispielsweise Blutleukozyten (PBLs), Monozyten, Nierenzellen oder Makrophagen und Zellen des immunologisch lymphatischen Systems.

Als mögliche Salze der Verbindungen der allgemeinen Formel I kommen vor allem Alkali-, Erdalkali- und Ammoniumsalze der Phosphatgruppe in Frage. Als Alkalisalze sind Lithium-, Natrium- und Kaliumsalze bevorzugt. Als Erdalkalisalze kommen insbesondere Magnesium- und Calciumsalze in Frage. Unter Ammoniumsalzen werden erfindungsgemäß Salze verstanden, die das Ammoniumion enthalten, das bis zu vierfach durch Alkylreste mit 1-4 Kohlenstoffatomen und/oder Aralkylreste, bevorzugt Benzylreste, substituiert sein kann. Die Substituenten können hierbei gleich oder verschieden sein.

Die Verbindungen der allgemeinen Formel I können basische Gruppen, insbesondere Amino-Gruppen enthalten, die mit geeigneten anorganischen oder organischen Säuren in Säureadditionssalze überführt werden können. Als Säuren kommen hierfür beispielsweise in Betracht: Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure.

Neben den Verbindungen der allgemeinen Formel I betrifft diese Anmeldung auch deren Tautomere und deren physiologisch verträgliche Salze anorganischer und organischer Säuren bzw. Basen, sowie Verfahren zu ihrer Herstellung und diese Verbindungen enthaltende Arzneimittel.

Da die Verbindungen der allgemeinen Formel I asymmetrische Kohlenstoffatome enthalten, sind auch sämtliche optisch aktiven Formen und racemische Gemische dieser Verbindungen Gegenstand der vorliegenden Erfindung.

Gegenstand dieser Anmeldung sind auch neue Liponucleotide. Die Darstellung dieser Konjugate wird exemplarisch in Beispielen 14-16 gezeigt. Das LCE spaltet auch 2-Chlor-2'-desoxy-adenosin-Konjugate (Cladribin-Konjugate) der Formel V, 9-(β-D-Arabinofuranosyl)-2-fluoradenin-Konjugate (Fludarabin-Konjugate) der Formel VI, 3-(2-Desoxy-β-D-erythro-pentofuranosyl)-3, 6, 7, 8-tetrahydroimidazo [4, 5-d] [1, 3] diazepin-8-ol-Konjugate (Pentostatin-Konjugate) der Formel VII. Die genannten Verbindungen lassen sich therapeutisch besser anwenden als die entsprechenden Nucleoside allein, da sie eine sehr gute Wirksamkeit und eine große therapeutische Breite zeigen und die gleichen Vorteile haben, wie die oben genannten Derivate der Formel I:

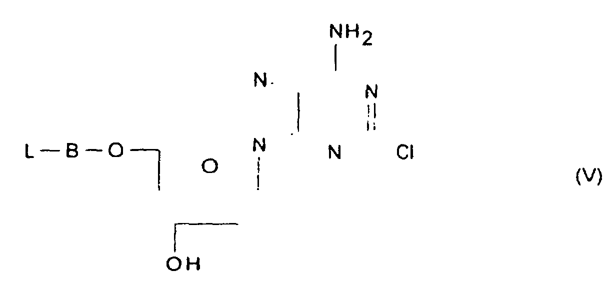

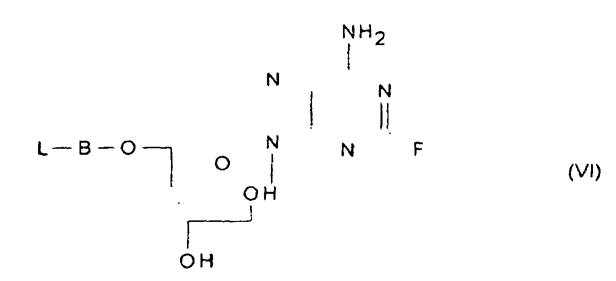

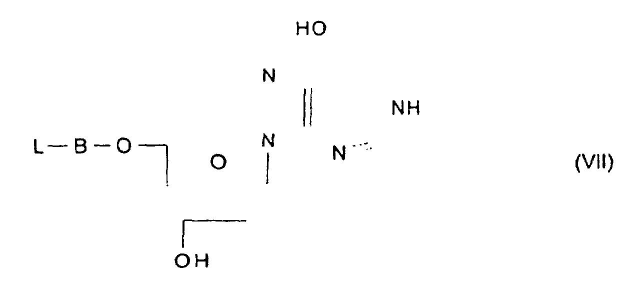

### Beispiel 1:

### Enzymatische Spaltung von AZT-DMDOPE

10 mg (3'-Desoxy-3'-azidothymidin)-5'-phosphorsäure-(3-dodecyl-mercapto-2-decyl-oxy)-propylester (AZT-DMDOPE) werden in 0,5 ml 0,1 M Tris-Pufferlösung suspendiert und nach Zugabe des Enzyms (0,5 mg bei Phosphodiesterase und 0,1 mg bei Phospholipase/Nuclease) 15 Stunden bei 37 °C inkubiert. Anschließend wird die Lösung dünnschichtchromatographisch untersucht. Nach Zugabe von 5 Tropfen gesättigter NaHCO₃-Lösung wurde weitere 6 Stunden bei 37 °C inkubiert und anschließend die Lösung dünnschichtchromatographisch untersucht.

Durch Dünnschichtchromatographie mit verschiedenen Elutionsmitteln wurde die Lösung hinsichtlich der Bildung der möglichen Spaltprodukte (AZT, AZT-monophosphat, DMDOP und Lipidmonophosphat) untersucht.

### Folgende Enzyme wurden in dem oben genannten Test eingesetzt:

a) Phospholipase C aus Bacillus cereus, 2000 U/0,5 ml, Boehringer Mannheim GmbH
b) Phospholipase D, Typ I, aus Kohl, 150 - 300 U/mg, Sigma
c) Phosphodiesterase aus Kalbsmilz, 2 U/mg, Boehringer Mannheim GmbH
d) Phosphodiesterase aus Schlangengift, Boehringer Mannheim GmbH
e) Nuclease aus Staphylococcus aureus, 15 000 U/mg, Boehringer Mannheim GmbH
f) LCE (Lipid-Cleavage-Enzyme)

### Ergebnis:

Aus der folgenden Tabelle geht hervor, daß nur im Falle der Phospholipase D eine schwache Fluoreszenzauslöschung auf der Höhe des Lipidphosphats festgestellt wird. In keinem der als Referenzenzyme verwendeten Fälle a) - e) konnte die Bildung von AZT oder AZP-monophosphat nachgewiesen werden.

Auß dem unveränderten AZT-DMDOPE konnte bei diesen Versuchen kein zusätzlicher Fleck im Rahmen der Nachweisgrenze gefunden werden.

| Enzym | Fluoreszenzauslöschung DC |
|---|---|
| Phospholipase C | negativ |
| Phospholipase D | schwach |
| Phosphodiesterase aus Kalbsmilz | negativ |
| Phosphodiesterase aus Schlangengift | negativ |
| Nuclease aus Staphylococcus aureus | negativ |
| LCE | positiv |

### Beispiel 2:

### Kommentar zu den Abbildungen

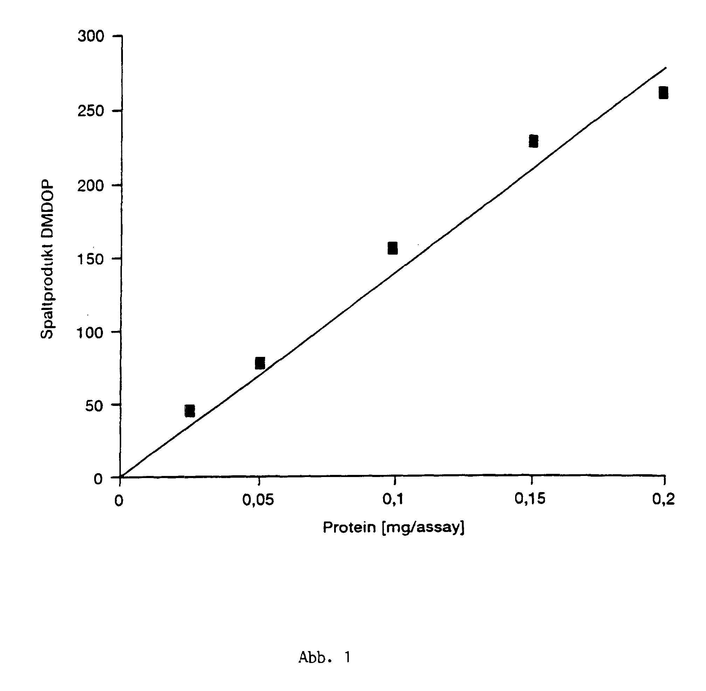

- Abb. 1:: Diese Abbildung zeigt die Aktivität des LCE, das aus der Membranfraktion von humanen, PHA-stimulierten peripheren Blutleukozyten erhalten wurde, in Abhängigkeit von der Proteinkonzentration. Es resultiert ein linearer Zusammenhang zwischen der Proteinkonzentration und der Menge des gebildeten Spaltproduktes DMDOP.

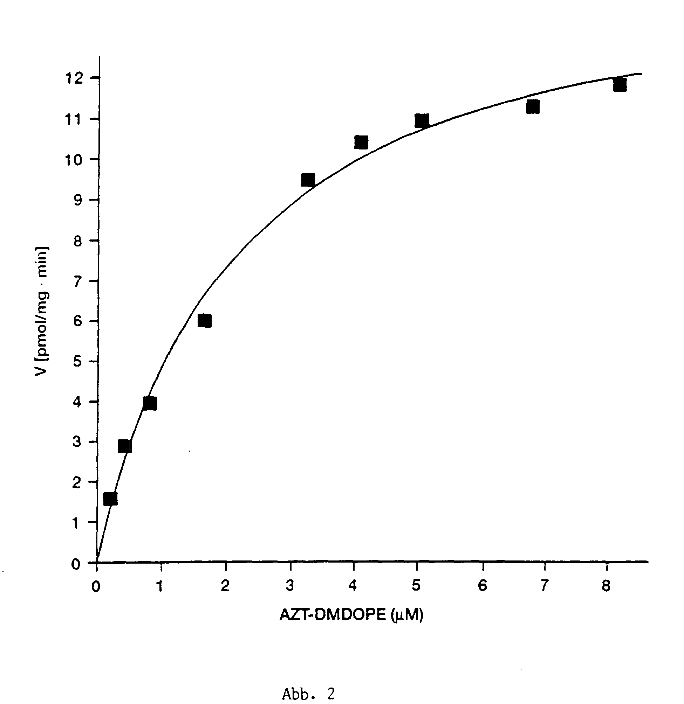

- Abb. 2.:: Diese Abbildung zeigt die Substratkinetik (Michaelis-Menten-Kinetik) des LCE, das aus der Membranfraktion von humanen, PHA-stimulierten peripheren Blutleukozyten erhalten wurde.

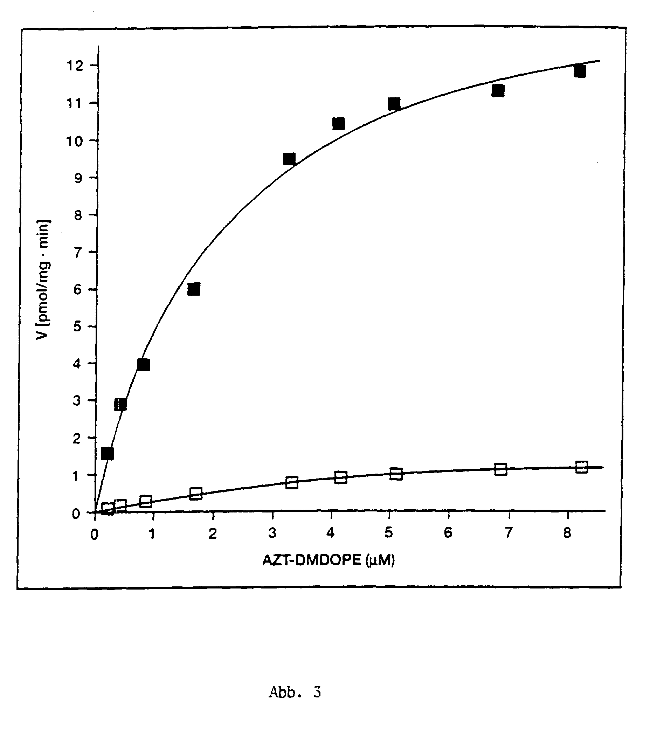

- Abb. 3:: Diese Abbildung zeigt die Substratkinetik (Michaelis-Menten-Kinetik) des LCE, das aus der Membranfraktion von humanen, PHA-stimulierten peripheren Blutleukozyten [■] und aus ruhenden (nicht stimulierten) peripheren Blutleukozyten [□] erhalten wurde.
- Abb. 4:: Diese Abbildung zeigt die Abhängigkeit der spezifischen Enzymaktivität des LCE, das aus der Membranfraktion von humanen, PHA-stimulierten peripheren Blutleukozyten erhalten wurde, von der Calciumionenkonzentration.

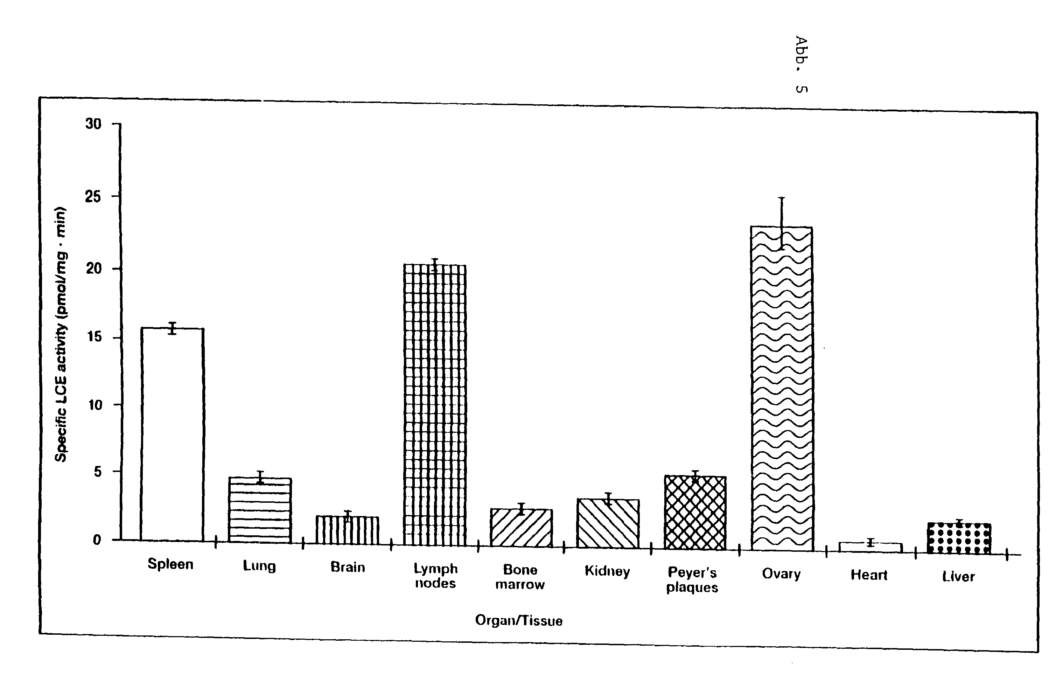

- Abb. 5:: Diese Abbildung zeigt die organ- bzw. gewebsspezifische Verteilung des LCE von Zellmembranpräparationen am unbehandelten Hund.
- Abb. 6:: Intrazelluläre Konzentrationen an AZT und AZT-Nukleotiden in stimulierten humanen PBL nach 1, 3, 6 und 24 h Inkubation mit:
(A) 00.3 µg AZT/ml (■) bzw. 1 µg AZT-DMDOPE/ml (□)
(B) 0.3 µg AZT/ml (■) bzw. 10 µg AZT-DMDOPE/ml (□)
(Mittelwerte, n = 2 Bestimmungen)
- Abb.7:: Intrazelluläre Konzentrationen an AZT und AZT-Nukleotiden in nichtstimulierten humanen PBL nach 1, 3, 6 und 24 h Inkubation mit:
(A) 00.3 µg AZT/ml (■) bzw. 1 µg AZT-DMDOPE/ml (□)
(B) 0.3 µg AZT/ml (■) bzw. 10 µg AZT-DMDOPE/ml (□)
(Mittelwerte, n = 2 Bestimmungen)
- Abb. 8:: Intrazelluläre Konzentrationen an AZT bzw. AZT-Nukleotiden in stimulierten humanen PBL nach 6 und 24 h Inkubation mit
1 µg AZT-DMDOPE /ml (A) bzw. 10 µg AZT-DMDOPE /ml (B):
■ Behandlung mit alkalischer Phosphatase
□ keine Behandlung mit alkalischer Phosphatase
(Mittelwerte, n = 2 Bestimmungen)
- Abb. 9:: Intrazelluläre Konzentration an AZT und AZT-Nukleotiden in P3X63Ag8.653-Zellen nach 6, 24 und 48 h Inkubation mit:
(A) 00.3 µg AZT/ml (■) bzw. 1 µg AZT-DMDOPE /ml (□)
(B) 0.3 µg AZT/ml (■) bzw. 10 µg AZT-DMDOPE /ml (□)
(Mittelwerte, n = 2 Bestimmungen)
- Abb. 10:: Intrazelluläre Konzentrationen an AZT bzw. AZT-Nukleotiden in P3X63Ag8.653-Zellen nach 6 bzw. 24 h Inkubation mit 1 µg BM 21.1290 Na/ml (A) bzw. 10 µg AZT-DMDOPE/ml (B):
■ Behandlung mit alkalischer Phosphatase
□ keine Behandlung mit alkalischer Phosphatase
(Mittelwerte, n = 2 Bestimmungen)
- Abb. 11:: Intrazelluläre Abklingkinetik von AZT und AZT-Nukleotiden in stimulierten humanen PBL nach 24 h Inkubation mit 0.3 µg AZT/ml /A) bzw. 10 µg AZT-DMDOPE /ml (B) Intrazelluläre Konzentration an AZT und AZT-Nukleotiden 1, 3, 6, 24 und 48 h nach Entfernung der AZT- bzw. AZT-DMDOPE-Inkubationslösung (Mittelwerte, n = 2 Bestimmungen)
- Abb. 12:: Enymatische Spaltung von [¹⁴C]-AZT-DMDOPE durch Membranfraktionen (100 µg Protein/Ansatz) stimulierter humaner PBL. DC-Chromatogramm der n-Heptan-Phase nach Adsorption des Substrates an Kieselgel 60 H und Trennung im IBA-System (2-Propanolol:n-Butylacetat:Aqua bidest., 10:6:4, v/v/v) mit Kieselgel 60 als stationärer Phase
- Abb.13:: Spezifische Aktivität [pmol mg-1 min-1] des LCE in Zellhomogenaten, Zytosol und Membranfraktionen stimulierter humaner PBL (Mittelwert ± SD, n = 6 Bestimmungen)
- Abb. 14:: Spaltung von AZT-DMDOPE durch die Membranfraktionen stimulierter humaner PBL in Abhängigkeit von der Proteinkonzentration (Mittelwert ± SD, n = 3 Bestimmungen)
- Abb. 15:: Spaltung von AZT-DMDOPE durch die Membranfraktion humaner Monozyten/Makrophagen (stimulierte Monozyten) in Abhängigkeit von der Proteinkonzentration (Mittelwert, n = 2 Bestimmungen)
- Abb. 16:: Spezifische Aktivität des LCE durch Membranfraktionen stimulierter und nichtstimulierter humaner PBL sowie humaner Monozyten und Monozyten/Makrophagen (stimulierte Monozyten) (humane PBL: Mittelwert ± SD, n = 6 Bestimmungen) (humane Monozyten: Mittelwert ± SD, n = 4 Bestimmungen)
- Abb. 17:: Spaltung von AZT-DMDOPE durch Membranfraktionen stimulierter humaner PBL in Abhängigkeit von der Inkubationszeit (Mittelwert ± SD, n = 3 Bestimmungen)
- Abb. 18:: DC-Chromatogramm von [¹⁴C]-AZT-DMOPE (A) und von [¹⁴C]-AZT-DMDOPE nach enzymatischer Spaltung durch Zellhomogenate von 5 x 10⁷ CEM-SS-Zellen (B) nach einer Inkubation von 6 h bei 37 °C. Extraktion der Thioetherlipide mit Diethylether:2-Propanol (9:1, v/v) und Trennung im IBAE-System (2-Propanol:n-Butylacetat:Aqua bidest:Eisessig, 3:5:1:1, v/v/v/v) mit Kieselgel 60 als stationärer Phase

### Beispiel 3:

### Einfluß von verschiedenen Enzyminhibitoren oder -aktivatoren auf die LCE-Aktivität

| Inhibitor/Aktivator | Angriffspunkt der Inhibierung/ Aktivierung | LCE Membranfraktion | Einfluß auf LCE-Aktivität |
|---|---|---|---|
| SQ 22536 | Adenylat-Cyclase | Niere (Balb/c) | keine |
| 7-Nitroindazol | NO-Synthese | Niere (Balb/c) | keine |
| RHC-80267 | DAG-Lipase | Niere (Balb/c) | keine |
| Neomycin | PLC, (PLD) | Niere (Balb/c) | keine |
| Wortmannin | PLD, (PLC) | Niere (Balb/c) | keine |
| Acetylsalicylsäur e | PLC | Niere (Balb/c) | keine |
| GTP-β-S | Gp Aktivator | Niere (Balb/c) | keine |
| D 609 | PLC | Niere (Balb/c) CEM-SS | Stimulation Stimulation |

### Beispiel 4:

### Subzelluläre Fraktionierung

### a. Herstellunq von Zellhomogenaten, Membran- und Zytosolfraktionen

Die zur Herstellung von Membran- und Zytosolfraktionen kultivierten Zellen wurden in Polypropylenröhrchen (50 ml) überführt und in der Minifuge T bei Raumtemperatur mit 1600 rpm für 10 min sedimentiert. Zur Entfernung von Resten des Kulturmediums wurde das Zellsediment dreimal mit kaltem PBS gewaschen und in Aufschlußpuffer mit einer Zelldichte von 1 - 5 x 10⁸ Zellen/ml aufgenommen. Die Zellsuspension wurde anschließend in einen auf Eis gekühlten Glashomogenisator überführt. Durch mehrmaliges Bewegen eines Teflonkolbens wurden die Zellen mechanisch aufgeschlossen, wobei der Zellaufschluß im Umkehr-Phasenkontrast-Mikroskop optisch kontrolliert wurde. Die aufgeschlossenen Zellen wurden anschließend zur Entfernung der Zellkerne und der nicht aufgeschlossenen Zellen in der Minifuge T bei 4 °C mit 1700 rpm für 10 min zentrifugiert. Der Überstand wurde vorsichtig mit einer Pipette abgehoben und mit 50 mM Tris-Puffer (pH 7.5) auf 10 % (w/v) Saccharose verdünnt. Das Zellhomogenat wurde nach Portionierung bei - 70 °C gelagert oder zur Isolierung von Membran- und Zytosolfraktionen herangezogen. Hierfür wurden 3.2 ml des Zellhomogenates in auf Eis gekühlte, dickwandige Polycarbonatröhrchen (3.2 ml) überführt und in der Beckmann-Tabletop-Ultrazentrifuge mit TLA-100.4 Rotor für 1 h bei 4 °C und 75000 rpm zentrifugiert. Die Überstände wurden vorsichtig mit einer Combitip-Pipette abgehoben und die gut sichtbaren Membransedimente mit jeweils 1 ml 50 mM Tris-Puffer (pH 7.5)/10 % Saccharose versetzt. Das Membransediment wurde mit Hilfe einer Spritze (5 ml) mit Kanüle (0.9 x 40 mm) grob homogenisiert. Die Grobhomogenate wurden vereinigt und unter Verwendung von Kanülen kleineren Durchmessers (0.8 x 40 mm und 0.45 x 25 mm) fein homogenisiert. Die Membran- und Zytosolfraktionen wurden nach Portionierung bei - 70 °C gelagert.

| | |
|---|---|
| Aufschluß-Puffer | 50 mM Tris pH 7.5 70 % (w/v) Saccharose 50 µg/ml APMSF |
| | |
| | Zum Lösen der Saccharose muß der Puffer unter Rühren leicht erwärmt werden. |

### b. Isolierung der Plasmamembran stimulierter humaner peripherer Blutlymphozyten (PBL) (Record et al. (1985), Biochem. Biophys. Acta 8/9 1-9)

Humane PBL wurden durch Zentrifugation im isotonischen Dichtegradienten isoliert und mit PHA-M 72 h stimuliert. Zur Entfernung von Resten des Kultivierungsmediums wurden die Zellen dreimal mit kaltem PBS gewaschen und nach Bestimmung der Zellzahl in flüssigem N₂ eingefroren und anschließend bei - 70 °C gelagert. Die Lyse der Zellen erfolgte durch dreimaliges Auftauen und Einfrieren in Puffer 1, wobei eine Zelldichte von 2.5 x 10⁸ Zellen/ml eingestellt wurde. In Ti60-Zentrifugenröhrchen (30 ml) wurde eine Mischung aus 11 ml Percoll und 2.13 ml Puffer 2 vorgelegt, welche zuvor mit 70 µl 2 N NaOH auf pH 9 eingestellt wurde. Zur Isolierung der Plasmamembran wurden 4 ml des Homogenates auf die Mischung aufgetragen und anschließend im Ti60-Rotor bei 4 °C und 39000 rpm für 10 min mit ausgeschalteter Bremse in der Beckmann-Ultrazentrifuge L 5 50 fraktioniert. Aus der oberen Phase der Zentrifugenröhrchen wurden 1 ml Fraktionen entnommen und mit 2 ml Puffer 3 verdünnt. Die Lösungen wurden anschließend in Ti50-Röhrchen (10.4 ml) überführt und zur Entfernung von Resten des Trennmediums in der Beckmann-Ultrazentrifuge L 5 50 mit Ti50-Rotor bei 4 °C und 45000 rpm für 45 min zentrifugiert. Die Überstände wurden mit einer Pipette abgehoben, fraktioniert, in flüssigem N₂ eingefroren und bei - 70 °C gelagert. Die Charakterisierung der Fraktionen erfolgte durch Bestimmung des Proteingehaltes und der Enzymaktivität der alkalischen Phosphatase.

| | | |
|---|---|---|
| Puffer 1 | 100 mM | KCl |
| | 5 mM | MgCl₂ x 6 H₂O |
| | 1 mM | ATP |
| | 2 mM | (4-Amedinophenyl)-methansulfonylfluorid (APMSF) |
| | 25 mM | Tris pH 9.6 |

| | | |
|---|---|---|
| Puffer 2 | 400 mM | KCI |
| | 20 mM | MgCl₂ x 6 H₂O |
| | 400 mM | Tris pH 9.6 |

| | | |
|---|---|---|
| Puffer 3 | 100 mM | KCI |
| | 5 mM | MgCl₂ x 6 H₂O |
| | 50 mM | Tris pH 7.42 |

### Beispiel 5:

### Herstellung von Substratlösungen für den LCE-Assay

Als Substrat wurde im LCE-Assay ein Gemisch aus [¹⁴C]-AZT-DMDOPE und nichtradioaktiv markiertem AZT-DMDOPE eingesetzt.

Ausgehend von einer [¹⁴C]-AZT-DMDOPE Stammlosung wurde durch Verdünnung mit Ethanol eine Lösung mit einer spezifischen Aktivität von 83.27 kBq/ml hergestellt. Die ethanolische Lösung konnte bei 4 °C unter Inertgas für 2 Monate gelagert werden.
In einem getrennten Ansatz wurde die nichtradioaktiv markierte Komponente des Substanzgemisches hergestellt. Hierfür wurde in Ethanol eine Lösung von AZT-DMDOPE mit einer Konzentration von 1 µmol/ml angesetzt und für 1 min im Eis/Wasserbad mit dem Ultraschallstab bei 30 % Energieabgabe kontinuierlich beschallt.

Zur Herstellung des Substratgemisches aus radioaktivem und nichtradioaktivem AZT-DMDOPE wurden aus diesen ethanolischen Lösungen pro Ansatz 1.67 kBq an radioaktiv markiertem [¹⁴C]-AZT-DMDOPE und 0.7 - 5 nmol der nichtmarkierten Verbindung in ein Glasröhrchen (10 ml) überführt. Das Lösungsmittel wurde im N₂-Strom evaporiert und AZT-DMDOPE in einem geeigneten Volumen an Aqua bidest. aufgenommen und für 1 min unter Kühlung im Eis/Wasserbad bei 30 % Energieabgabe kontinuierlich beschallt.

### Beispiel 6:

### Lipid-Cleavage-Enzyme (LCE)-Assay

Zur Untersuchung der enzymatischen Spaltung in Abhängigkeit von der Zeit und der Proteinkonzentration wurden üblicherweise 5 nmol AZT-DMDOPE und 0.98 nmol [¹⁴C]-AZT-DMDOPE (1.67 kBq) in einem Volumen von 200 µl Aqua bidest. im LCE-Assay eingesetzt. Das Pipettierschema ist in der folgenden Tabelle angegeben.

**Tabelle**

| **Pipettierschema für den LCE-Assay zur Bestimmung des Umsatzes von AZT-DMDOPE** | | |
|---|---|---|
| | **Reaktionsansatz** | **Kontrolle** |
| **EGTA [20 mM]** | **50** | **50** |
| **Tris, pH: 8.0 [1 M]** **[µl/Ansatz]** | **50** | **50** |
| **AZT-DMDOPE /[**^{**14**}**C]-AZT-DMDOPE** **[nmol/Ansatz]** **[µl/Ansatz]** | **0.10 - 5.98** **5 - 200** | **0.10 - 5.98** **5 - 200** |
| **Protein** **[mg/Ansatz]** **[µl/Ansatz]** | **0.0125 - 0.2** **x** | **-** **-** |
| **Aqua bidest.** **[µl]** | **ad 500** | **ad 500** |

Zur Untersuchung der enzymatischen Spaltung in Abhängigkeit von der Substratkonzentration wurde eine Substratstammlösung von 0.98 nmol [¹⁴C]-AZT-DMDOPE und 0.7 nmol AZT-DMDOPE/80 µl Aqua bidest. im LCE-Assay angesetzt. Die verschiedenen Substratkonzentrationen im LCE-Assay wurden durch kontinuierliche Verdopplung bzw. Halbierung des Volumens an Substratlösung eingestellt. Die Ansätze wurden in Glasröhrchen (5 ml) vorgelegt und durch Zugabe der Substratlösung gestartet. Die Inkubation wurde bei 37 °C im Wasserbad unter leichtem Schütteln durchgeführt.

### Beispiel 7:

### Extraktion von DMDOP aus wäßriger Matrix

Die Reaktionsansätze des LCE-Assays wurden nach entsprechender Inkubationszeit dem Wasserbad entnommen, und die Reaktion durch Zugabe von 750 µl 2-Propanol gestoppt. Nach gründlicher Durchmischung wurden die Ansätze anschließend mit 700 µl auf Raumtemperatur erwärmtem n-Heptan versetzt. Die Ansätze wurden erneut für 30 sec gut gemischt und zur Beschleunigung der Phasentrennung in der Minifuge T bei 3200 rpm für 15 min bei Raumtemperatur zentrifugiert. Anschließend wurden 500 µl der oberen Phase (n-Heptan) entnommen und in neue Glasröhrchen (5 ml) mit 10 mg Kieselgel 60 H und 200 µl n-Heptan überführt. Die Ansätze wurden für 30 sec gut durchmischt und zur Sedimentation des Kieselgels für 10 min unter oben genannten Bedingungen zentrifugiert. Vom Überstand wurden anschließend 500 µl entnommen und mit 3 ml Szintillationsflüssigkeit versetzt. Die Messung der Radioaktivität erfolgte für 4 min im Flüssigkeits-Szintillations-Analysator. Zur Bestimmung des Absolutwertes an Radioaktivität wurden 200 µl der Substratlösung mit 3 ml Szintillationsflüssigkeit versetzt und unter gleichen Bedingungen im Flüssigkeits-Szintillations-Analysator gemessen.

### Beispiel 8:

### In-vitro-Pharmakokinetikstudien mit AZT-DMDOPE und AZT

### a. Bestimmung der intrazellulären Konzentrationen an AZT und/oder AZT-Nukleotiden in stimulierten und nichtstimulierten humanen PBL nach Inkubation mit AZT-DMDOPE bzw. AZT

Zur Bestimmung der intrazellulären Konzentrationen an AZT und/oder AZT-Nukleotiden nach Inkubation mit AZT-DMDOPE bzw. AZT wurden humane PBL aus "Buffy coat" isoliert.

Hierfür wurde "Buffy-coat" von gesunden Spendern gemischt und durch Zentrifugation in einem isotonischen Medium mit einer Dichte von 1.077 g/ml fraktioniert. Da mononukleare Blutzellen (Monozyten und Lymphozyten) eine geringere Dichte als Erythrozyten und polymorphkernige Granulozyten haben, bilden sie einen Ring an der Grenze zwischen Trennmedium und Probe und können mit Hilfe einer Pipette abgenommen werden. Erythrozyten und polymorphkernige Granulozyten sedimentieren aufgrund ihrer höheren Dichte durch das Medium und lassen sich somit von PBL separieren.
Zur Stimulation der Zellproliferation wurde Phytohämaglutinin A (Mukoprotein) (PHA-M), der mitogene Lektinextrakt aus der roten Feuerbohne (Phaseolus vulgaris), eingesetzt. Phytohämagglutinin ist eine Familie von 5 Isolektinen, die als Tetramere vorliegen. Die Untereinheiten bestehen aus einem lymphozytenreaktiven Typ-L und einem erythrozytenreaktiven Typ-E. Typ-L hat eine hohe Affinität zu Lymphozytenoberflächenrezeptoren und ist somit verantwortlich für die mitogenen Eigenschaften. Die Stimulation der Lymphozyten wurde in KPMI 1640 Komplettmedium III für 72 h bei 37 °C und 5 % CO₂ durchgeführt. Nach Beendigung der Stimulation wurden zur Erhöhung der Zellzahl die Zellen für weitere 24 h in RPMI 1640 Komplettmedium IV kultiviert.

Zur Bestimmung der intrazellulären Konzentrationen an AZT und AZT-Nukleotiden wurden die Zellen in RPMI 1640 Komplettmedium II mit einer Dichte von 1 x 10⁶ Zellen/ml in Gewebekulturflaschen (50 ml/25 cm²) überführt, und in Anwesenheit von 0.03 bzw. 0.3 µg AZT/ml (Charge 15) und 1 bzw. 10 µg AZT-DMDOPE/ml für 1 - 24 h inkubiert.

Die Konzentrationen an AZT-DMDOPE und AZT orientierten sich hierbei an den IC50-Werten für die antiretrovirale Wirksamkeit, die für AZT und AZT-DMDOPE in HIV-1-infizierten humanen PBL ermittelt wurden.

Die Kulturen wurden anschließend für 1, 3, 6 und 24 h bei 37 °C und 5 % CO₂ inkubiert. AZT und seine Nukleotide wurden anschließend mit 60 % Methanol extrahiert. Die extrahierten Nukleotide wurden mit alkalischer Phosphatase quantitativ zu AZT dephosphoryliert, da nur AZT, nicht jedoch die entsprechenden Nukleotide mit Hilfe eines Radioimmuno-Assays detektiert werden kann.

Die Konzentrationen an intrazellulärem AZT, die mit Hilfe des Radioimmuno-Assays bestimmt wurden, setzten sich dementsprechend aus der des AZT und den der AZT-Nukleotide zusammen. Eine separate Quantifizierung der Konzentrationen an AZT-MP, AZT-DP und AZT-TP wurde nicht durchgeführt. Experimentelle Beobachtungen an stimulierten PBL konnten eine im Vergleich zu AZT-DP und AZT-TP um Faktor 40 höhere Konzentration an AZT-MP belegen (Arner et al., 1992, J. Biol. Chem. 267, 10968-10975). Entsprechend kann angenommen werden, daß sich die Nukleotidfraktion fast ausschließlich aus AZT-MP zusammensetzt.

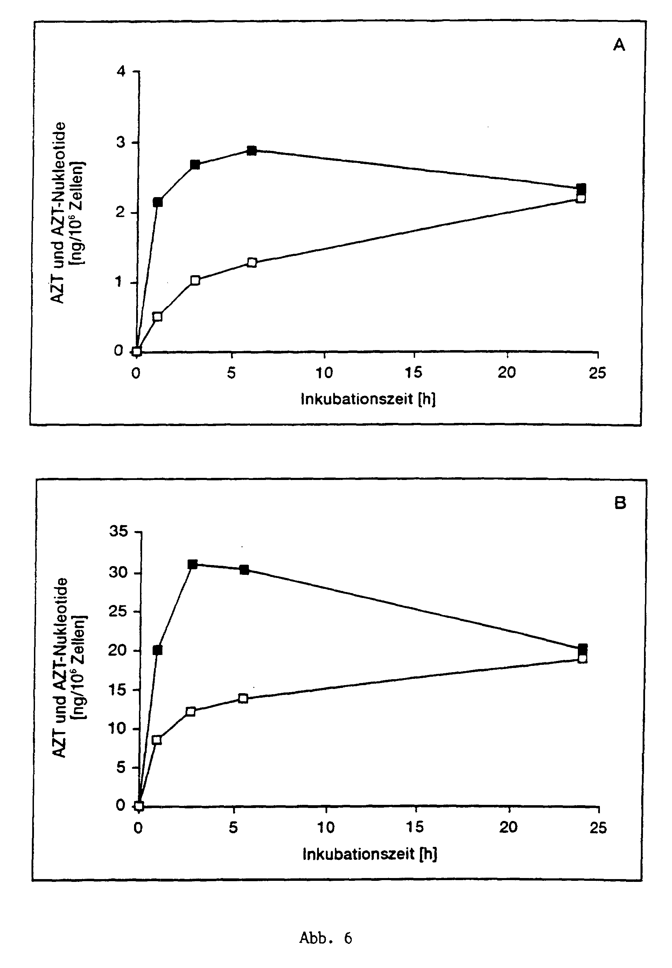

In stimulierten humanen PBL wurden bei Inkubation mit 0.03 bzw. 0.3 µ g AZT/ml maximale Konzentrationen von 2.90 bzw. 30.97 ng/10⁶ Zellen nach einer Inkubation von 6 bzw. 3 h erreicht (Abb. 6). Die Konzentrationen fielen anschließend bei zunehmender Inkubationszeit auf 2.32 bzw. 19.88 ng/10⁶ Zellen ab. Nach Inkubation mit 1 bzw. 10 µg AZT-DMDOPE/ml zeigte sich ein über die gesamte Inkubationszeit stetig steigender intrazellulärer Spiegel an AZT und seiner Nukleotiden Nach 24 h Inkubation mit AZT-DMDOPE und AZT waren die intrazellulären Spiegel identisch. In weiteren Experimenten konnte gezeigt werden, daß daß nach einer Inkubationszeit von 18 - 48 h die intrazellulären Konzentrationen an AZT und AZT-Nukleotiden nach Inkubation mit AZT-DMDOPE sogar größer sind als nach Inkubation mit der äquipotenten Konzentration an AZT.

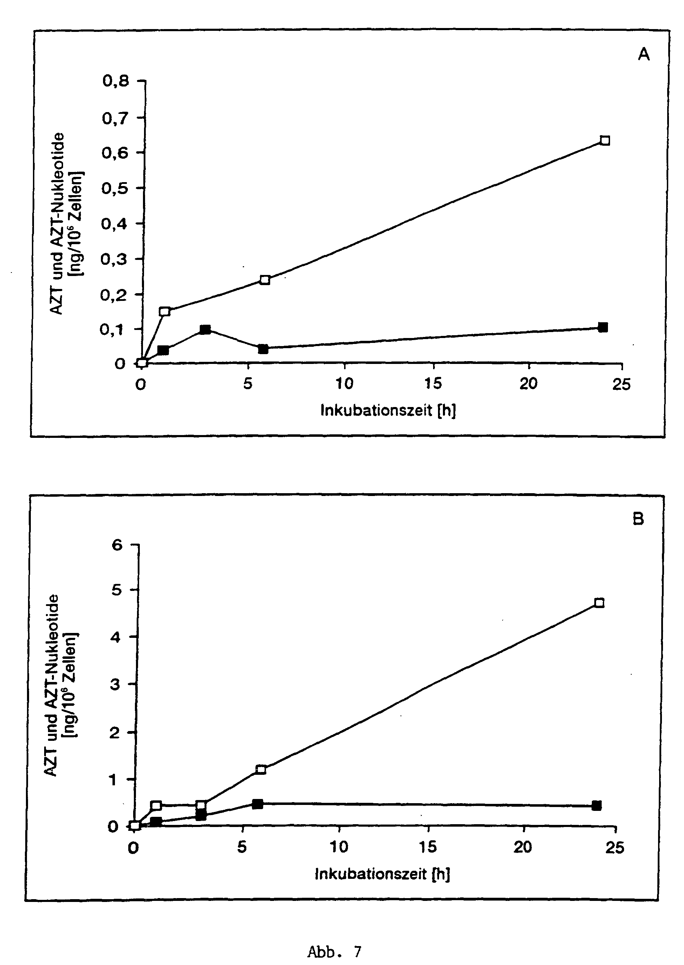

Bei nichtstimulierten humanen PBL kehrten sich die Verhältnisse um (Abb. 7). So waren die intrazellulären Konzentrationen an AZT und AZT-Nukleotiden nach Inkubation mit AZT-DMDOPE über die gesamte Inkubationszeit größer als nach Inkubation mit der äquipotenten Konzentration an AZT. Nach Inkubation mit 1 bzw. 10 µg AZT-DMDOPE/ml wurden maximale intrazelluläre Konzentrationen an AZT und AZT-Nukleotiden von 0.62 bzw.
4.74 ng/10⁶ Zellen erreicht, während nach Inkubation mit 0.03 bzw.
0.3 µg AZT/ml Konzentrationen von 0.10 bzw. 0.47 ng/10⁶ Zellen detektiert werden konnten.

Zur separaten Quantifizierung von AZT und den AZT-Nukleotiden nach Inkubation mit BM 21.1290 Na wurden alle intrazellulären Konzentrationen an AZT und AZT-Nukleotiden mit und ohne Behandlung mit alkalischer Phosphatase bestimmt. Diese Experimente wurden an stimulierten humanen PBL durchgeführt.

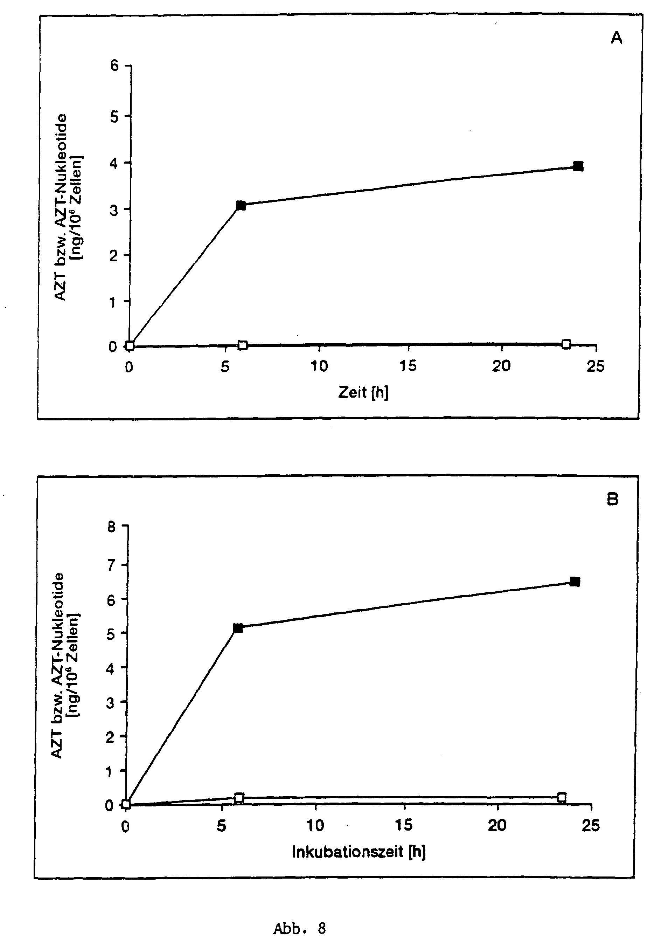

Hierfür wurden Zellsuspensionen mit einer Dichte von 10⁶ Zellen/ml mit 1 bzw. 10 µg AZT-DMDOPE/ml für 6 bzw. 24 h bei 37 °C und 5 % CO₂ inkubiert. Die maximalen intrazellulären Konzentrationen an freiem AZT nach Inkubation mit 1 bzw. 10 µg AZT-DMDOPE/ml wurden nach 6 bzw. 24 h mit 0.03 bzw.
0.15 ng/10⁶ Zellen detektiert (Abb. 8). Wurden nach Behandlung mit alkalischer Phosphatase zusätzlich die phosphorylierten intrazellulären AZT-Nukleotide erfaßt. so wurden nach 24 h maximale Konzentrationen von 3.85 bzw. 6.41 ng/10⁶ Zellen gefunden.

### b. Bestimmung der intrazellulären Konzentrationen an AZT und/oder AZT-Nukleotiden in P3X63Ag8.653-Zellen nach Inkubation mit AZT-DMDOPE bzw. AZT

Die Bestimmung der intrazellulären Konzentrationen an AZT und AZT-Nukleotiden in Thymidikinasedefizienten Zellen sollte weitere Erkenntnisse über die intrazelluläre Spaltung der Testsubstanz AZT-DMDOPE liefern. Intrazelluläres AZT kann in diesen Zellen aufgrund eines Mangels an Thymidin-Kinase (TK) nicht in ATZ-MP und somit in das therapeutisch wirksame AZT-TP überführt werden. Die Bestimmung der intrazellulären Konzentrationen an AZT und AZT-Nukleotiden nach Inkubation mit AZT-DMDOPE könnte somit einen weiteren Hinweis auf die Spaltung der Teststubstanz geben.

Hierfür wurden in Gewebekulturschalen (60 x 15 mm) 1 x 10⁷ Zellen in RPMI 1640 Komplettmedium I mit 0.03 bzw. 0.3 µg AZT/ml und 1 bzw. 10 µg AZT-DMDOPE/ml über 6, 24 und 48 h inkubiert. Nach Extraktion von AZT und der AZT-Nukleotide und einer Behandlung mit alkalischer Phosphatase wurde die Konzentration an AZT mit Hilfe des Radioimmuno-Assays bestimmt.

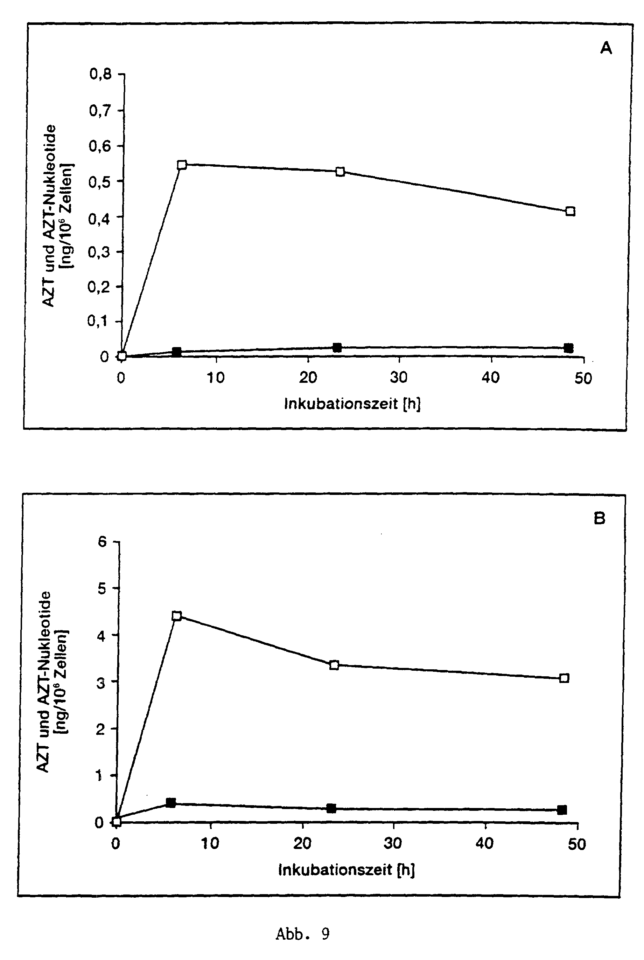

Der graphischen Auftragung der Versuchsergebnisse ist zu entnehmen, daß die intrazellulären Konzentrationen an AZT und AZT-Nukleotiden mit AZT-DMDOPE deutlich höher waren als nach Inkubation mit äquipotenten Konzentrationen an AZT. So wurden nach 6 h Inkubation mit 1 bzw. 10 µg AZT-DMDOPE/ml maximale Konzentrationen an AZT und AZT-Nukleotiden von 0.55 bzw. 4.40 ng/10⁶ Zellen erreicht (Abb. 9).

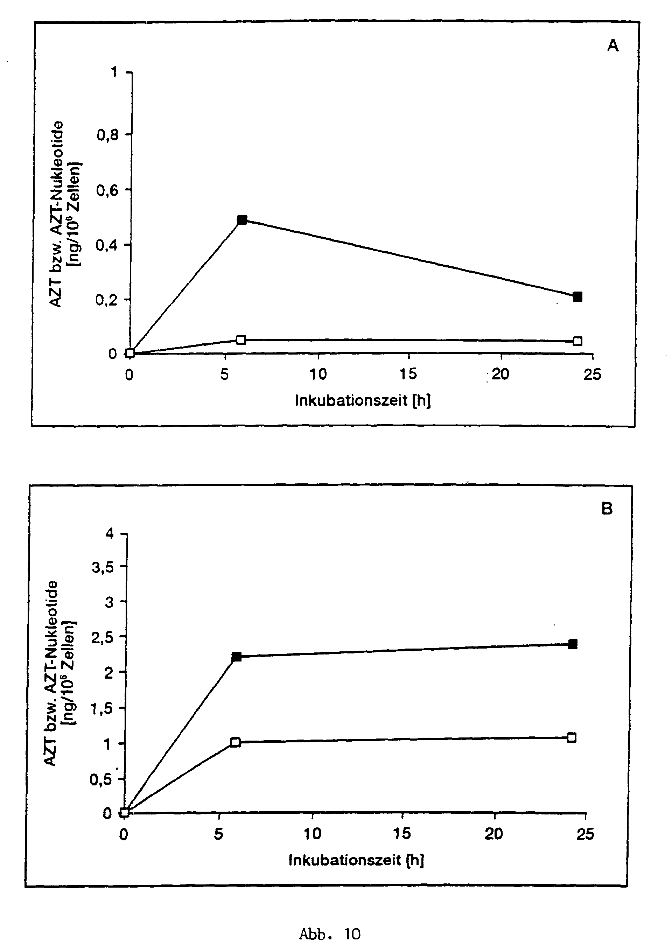

Die maximalen intrazellulären Konzentrationen an AZT und AZT-Nukleotiden nach Inkubation mit 0.03 bzw. 0.3 µg AZT/ml wurden nach 48 h mit 0.03 bzw. 0.31 ng/10⁶ Zellen bestimmt.
Zur Quantifzierung der Anteile an phosphoryliertem AZT wurden die intrazellulären Konzentrationen an AZT und AZT-Nukleotide vor und nach einer Behandlung der zellulären Extrakte mit alkalischer Phosphatase verglichen. Die Substanzspiegel an phosphoryliertem AZT nach Inkubation mit 1 bzw. 10 µg AZT-DMDOPE/ml ergaben hierbei Werte von 0.48 bzw.
2.42 ng/10⁶ Zellen (Abb. 10). Wurde nur AZT erfaßt, so wurden nach 24 h maximale Konzentrationen von 0.05 bzw. 1.10 ng/ml erreicht. Die Unterschiede in den intrazellulären Konzentrationen an AZT-Nukleotiden nach Inkubation mit AZT und AZT-DMDOPE können durch eine intrazelluäre Spaltung der Testsubstanz AZT-DMDOPE zu AZT-MP erklärt werden.

### c. Abklingkinetik von AZT und AZT-Nukleotiden in stimulierten humanen PBL nach Inkubation mit AZT-DMDOPE bzw. AZT

Stimulierte humane PBL wurden für 24 h mit 0.3 µg AZT/ml bzw. 10 µg AZT-DMDOPE/ml inkubiert. Anschließend wurden 1 x 10⁷ Zellen in Gewebekulturflaschen (50 ml/25 cm²) mit RPMI 1640 Komplettmedium I überführt. Nach 1, 3, 6, 24 und 48 h wurde AZT und die AZT-Nukleotide aus der zellulären Matrix extrahiert. Nach einer Behandlung mit alkalischer Phosphatase wurde mit Hilfe des Radioimmuno-Assays die Konzentration an AZT bestimmt.

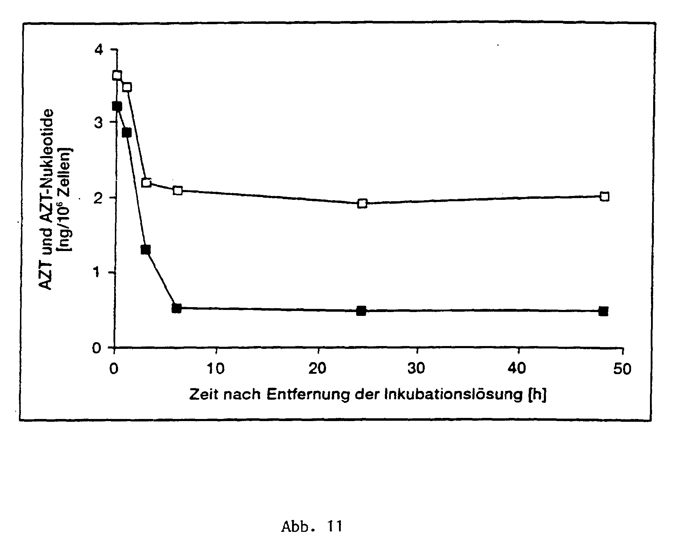

Der graphischen Darstellung der Versuchsergebnisse ist zu entnehmen, daß nach Inkubation der Zellen mit AZT die intrazellulären Konzentrationen an AZT und AZT-Nukleotiden rasch abfallen und nach 6 h einen konstanten Wert von 0.40 ng/10⁶ Zellen erreichen. Bei Inkubation mit einer äquipotenten Konzentration an AZT-DMDOPE hingegen nehmen die AZT bzw. AZT-Nukleotide weniger stark ab und erreichen nach 3 h bei einer wesentlich höheren Konzentration einen über 48 h konstanten Wert von 1.80 ng/10⁶ Zellen (Abb. 11). Da nichtphosphoryliertes AZT durch mehrmaliges Waschen mit PBS aus der Zelle entfernt wurde sind die intrazellulären Konzentrationen hauptsächlich auf phosphorylierte AZT-Nukleotide zurückzuführen. Eine Inkubation mit AZT-DMDOPE stellt somit, im Vergleich zu einer aquipotenten Konzentration an AZT, der Zelle eine um Faktor 4.5 höhere Konzentration an phosphoryliertem AZT zur Verfügung. Diese großen Unterschiede in den zellulären Konzentrationen an AZT-Nukleotiden nach Inkubation mit AZT-DMDOPE und AZT können durch eine direkte intrazelluläre Spaltung des Thioetherlipid-AZT-Konjugates AZT-DMDOPE zu AZT-MP und dem korrespondierenden Thioetherlipidteil DMDOP erklärt werden.

### Beispiel 9:

### Charakterisierung des AZT-DMDOPE - spaltenden Enzyms/Enzymsystems (LCE)

Untersuchungen zur enzymatischen Spaltung von AZT-DMDOPE durch Zellhomogenate humaner PBL und CEM-SS-Zellen konnten zeigen, daß AZT-DMDOPE zu DMDOP und AZT-MP metabolisiert wird. Die Charakterisierung des LCE war Ziel der folgenden Untersuchungen. So wird die enzymatische Spaltung von AZT-DMDOPE in Abhängigkeit von der Proteinkonzentration, der Inkubationszeit und zweiwertiger Metallkationen untersucht. In Abhängigkeit verschiedener Substratkonzentrationen wird in enzymkinetischen Untersuchungen die apparenten Michaelis-Menten-Parameter K_{M} und vₘₐₓ bestimmt.

Für diese Untersuchungen ist ein Enzym-Assay etabliert, in dem AZT-DMDOPE und [¹⁴C]-AZT-DMDOPE als Substrat eingesetzt wurde (Beispiel 6). Bei proteinund zeitabhängigen Messungen wurden 5 nmol AZT-DMDOPE und 0.98 nmol (1.67 kBq) [¹⁴C]-AZT-DMDOPE /Ansatz verwendet. Bei substratabhängigen Umsetzungen wurde eine Stammlösung von 0.98 nmol [¹⁴C]-AZT-DMDOPE und 0.7 nmol AZT-DMDOPE /80 µl eingesetzt. Die verschiedenen Substratkonzentrationen wurden anschließend durch kontinuierliche Verdopplung bzw. Halbierung des Volumens an Substratlösung eingestellt.

Als Enzymquelle können z. B. Zellhomogenate, Zytosol- und Membranfraktionen bekannter Proteinkonzentrationen von verschiedenen humanen Zellen eingesetzt werden.

### a. Isolierung und Quantifizierung von DMDOP nach enzymatischer Spaltung von AZT-DMDOPE

Die Quantifizierung der enzymatischen Spaltung von AZT-DMDOPE wurde durch eine zweistufge Extraktion des Metaboliten DMDOP mit n-Heptan und anschließender Adsorption von Resten des Substrates AZT-DMDOPE an Kieselgel 60 H durchgeführt. Hierbei wurden die Reaktionsansätze mit 2-Propanol und n-Heptan versetzt. wobei sich das im Vergleich zum Substrat unpolare DMDOP in der n-Heptan-Phase anreichert. Die n-Heptan-Phase wurde dann mit Kieselgel 60 H versetzt, wobei Reste des Substrates AZT-DMDOPE adsorbiert werden. Die n-Heptan-Phase wurde anschließend durch Zentrifugation vom Kieselgel separiert, in 3 ml Aqua luma überführt und die in ihr enthaltene Menge an radioaktiv markiertem DMDOP im Flüssigkeits-Szintillations-Analysator über einen Zeitraum von 5 min bestimmt. Aus diesen Ergebnissen konnte die prozentuale Umsetzung des Substrates AZT-DMDOPE zu DMDOP und die spezifische Aktivität des LCE errechnet werden. Als spezifische Aktivität wurde hierbei die Menge an DMDOP bezeichnet die pro 1 mg Protein der eingesetzten Zellpräparationen pro Minute gebildet wurde.

Zur Überprüfung der selektiven Extraktion von DMDOP aus dem Reaktionsgemisch wurden Optimierungsexperimente mit Membranfraktionen stimulierter humaner PBL durchgeführt. Die Proteinkonzentration war hierbei 100 µg/Ansatz. Parallel wurden Ansätze ohne Protein mitgeführt. Nach der Extraktion wurde die n-Heptan-Phase im N₂-Strom evaporiert, und die Rückstände in einem Gemisch aus Methanol und Essigsäureethylester (1:1, v/v) aufgenommen.

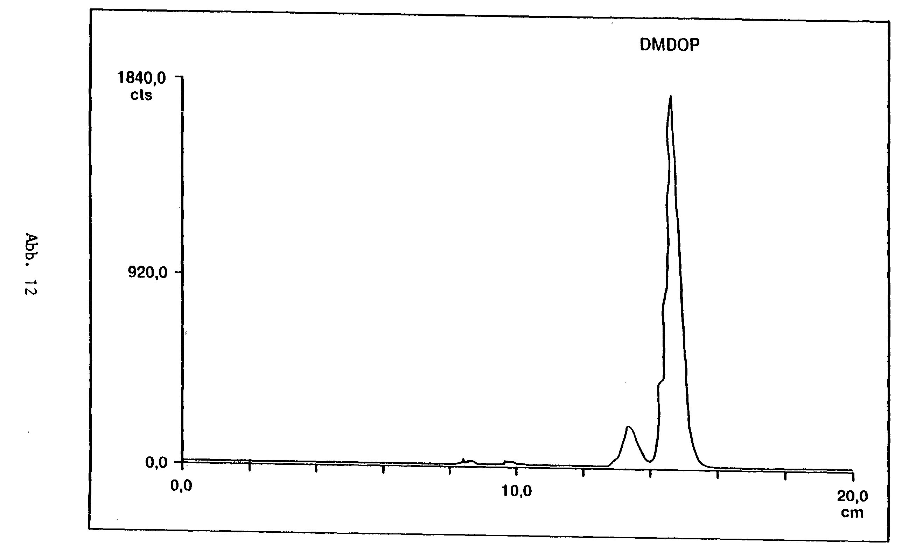

Die dünnschichtchromatographische Analyse erfolgte im IBA-System. Der Reaktionswert ließ hierbei nur den Peak der Muttersubstanz [¹⁴C]-AZT-DMDOPE erkennen. Im Reaktionsansatz wurde ein Substanzpeak am Ende der Laufmittelfront detektiert, der aufgrund des R_{f}-Wertes eindeutig als der Metabolit [¹⁴C]-DMDOP identifiziert werden konnte (Abb. 12). Der Peak, der unmittelbar vor dem DMDOP-Substanzpeak läuft, kann keiner der bekannten Verbindungen zugeordnet werden. Vermutlich handelt es sich hierbei um eine Verbindung, die durch Oxidation des Schwefels im Thioetherlipidteil von DMDOP entstanden ist. Durch eine Extraktion mit n-Heptan konnte somit eine Isolation des enzymatischen Spaltproduktes DMDOP aus dem Enzym-Assay durchgeführt werden. Eine einfache und schnelle Quantifizierung des aus AZT-DMDOPE freigesetzten Metaboliten DMDOP war somit gewährleistet.

### b. Spaltung von AZT-DMDOPE durch Zellhomogenate, Membran- und Zytosolfraktionen stimulierter humaner PBL

In einer ersten Versuchsreihe wurde die enzymatische Spaltung von AZT-DMDOPE durch Zellhomogenate sowie durch Zytosol- und Membranfraktionen stimulierter humaner PBL untersucht.

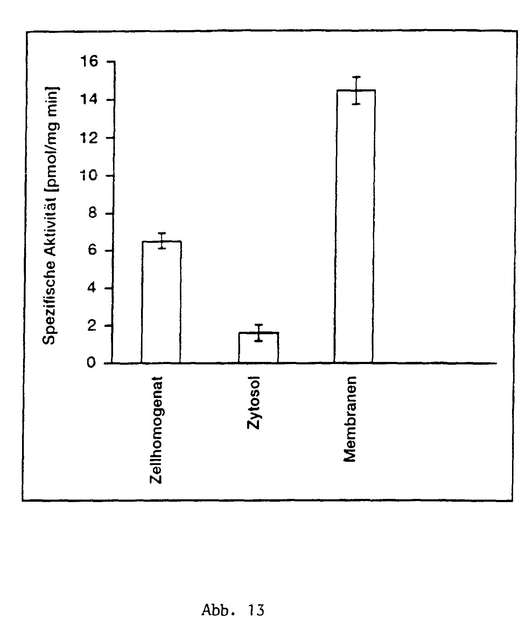

Nach Bestimmung der Proteinkonzentration in Zellhomogenaten, Zytosol- und Membranfraktionen mit Hilfe des Bicinchoninsäure(BCS)-Tests wurden 0.025 - 0.20 mg Protein/Ansatz eingesetzt. Die Reaktionsansätze wurden 2 h bei 37 °C inkubiert, anschließend das Produkt DMDOP durch Extraktion mit n-Heptan aus den Ansätzen isoliert und die spezifische Aktivität des LCE bestimmt (Abb. 13).

Die AZT-DMDOPE-spaltende Aktivität war im Zellhomogenaten und in den Zytosolfraktionen mit 6.48 ± 0.38 (n = 6) und 1.65 ± 0.40 pmol mg⁻¹ min⁻¹ (n = 6) um Faktor 1.59 bzw. 6.3 kleiner als die spezifische Aktivität in den Membranfraktionen mit 14.23 ± 0.70 (n = 6) pmol mg⁻¹ min⁻¹. Bei Isolierung der Membranfraktionen kommt es offensichtlich zu einer Anreicherung des LCE. Basierend auf diesen Ergebnissen wurden in den sich anschließenden Experimenten zur Bestimmung der enzymatischen Parameter des LCE Membranfraktionen eingesetzt.

Die Membranfraktion, die durch den mechanischen Aufschluß der Zellen und anschließender Ultrazentrifugation des Zellhomogenates gewonnen wurde, stellt ein Gemisch aus Fragmenten der Plasma- und Kernmembran sowie der Membranen der Zellorganellen dar.

Eine Differenzierung der verschiedenen Membranfragmente war durch Ultrazentrifugation eines Zellhomogenates im Percoll-Dichtegradienten und einer sich anschließenden Charakterisierung durch den Plasmamembranmarker alkalische Phosphatase möglich.

Zur Isolierung der Plasmamembranen wurden 2 ml einer Zellsuspension stimulierter humaner PBL mit einer Zelldichte von 2.5 x 10⁸ Zellen/ml durch dreimaliges Einfrieren und Auftauen aufgeschlossen und durch Zentrifugation im Percoll-Dichtegradienten fraktioniert. Dem Zentrifugat wurden 10 Fraktionen (1 ml) entnommen, Reste des Trennmediums entfernt und in den einzelnen Fraktionen die Aktivität der alkalische Phosphatase mit p-Nitrophenylphosphat als Substrat bei 305 nm gemessen.

Die höchste Aktivität an alkalischer Phosphatase war in Fraktion 8 zu bestimmen. Die Absorption der verbleibenden Fraktionen war wesentlich geringer.. Dies weist auf eine Anreicherung der Plasmamembranen in Fraktion 8 hin. In jeder Fraktion wurde nach Bestimmung der Proteinkonzentration die spezifische LCE-Aktivität ermittelt. Hierfür wurde eine Proteinkonzentration von 0.05 mg/Ansatz eingestellt und die Reaktionsansätze bei 37 °C für 2 h inkubiert. Die höchste spezifische LCE-Aktivität von 4.40 pmol mg⁻¹ min⁻¹ min konnte hierbei in Fraktion 8 bestimmt werden.

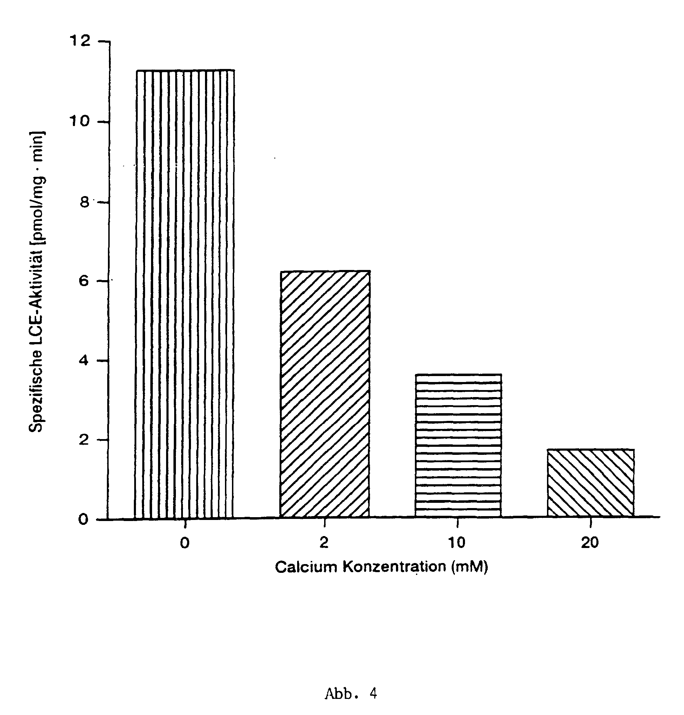

In allen weiteren Fraktionen wurden geringere spezifische Aktivitäten von 1.10 - 1.90 pmol mg⁻¹ min⁻¹ gemessen (Abb. 4:32). Die höchste spezifische Aktivität des LCE wurde somit in der Fraktion bestimmt, die gleichzeitig die höchste Aktivität an alkalischer Phosphatase aufweist. Dieser Befund zeigt somit das Vorkommen des LCE in der Fraktion mit dem höchsten Anteil an Fragmenten der Plasmamembran an.

### c. Abhängigkeit der spezifischen LCE-Aktivität von der Proteinkonzentration

In weiterführenden Untersuchungen sollte die Umsetzung von AZT-DMDOPE in Abhängigkeit steigender Proteinkonzentrationen der Zellpräparationen bestimmt werden.

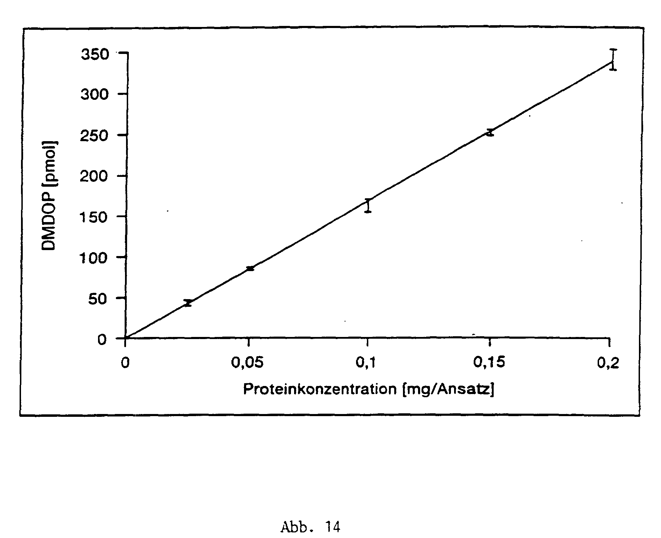

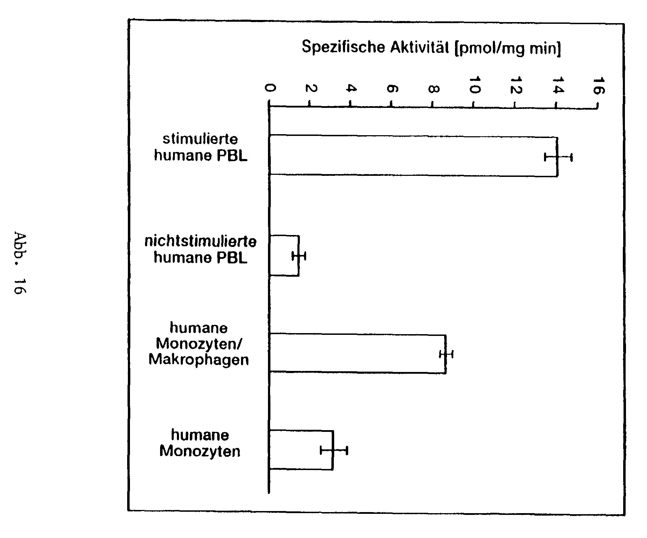

Hierbei wurden Membranfraktionen stimulierter und nichtstimulierter humaner PBL sowie humaner Blutmonozyten verwendet. Die Umsetzung von AZT-DMDOPE zu DMDOP durch die Membranfraktionen stimulierter humaner PBL wies beim Einsatz von 0.025 - 0.2 mg Protein/Ansatz lineares Verhalten auf (Abb. 14). Die spezifische LCE-Aktivität betrug 14.23 ± 0.7 pmol mg⁻¹ min⁻¹ (n = 6) (Abb. 16). Bei der Umsetzung von AZT-DMDOPE durch Proteine der Membranfraktion nichtstimulierter humaner PBL zeigte sich eine lineare Abhängigkeit nur im Konzentrationsbereich bis zu 0.05 mg Protein/Ansatz. Bei höheren Proteinkonzentrationen war keine Linearität mehr gegeben. Die spezifische LCE-Aktivität, die aus den Umsetzungen im linearen Bereich berechnet wurde, betrug 1.45 ± 0.32 pmol mg⁻¹ min⁻¹ (n = 6).

Zur Bestimmung der spezifischen LCE-Aktivität in humanen Blutmonozyten wurden diese im hypertonischen Dichtegradienten isoliert. Monozyten haben mit 1.068 g/ml im Durchschnitt eine etwas geringere Dichte als Lymphozyten mit 1.070 g/ml. Dieser Unterschied in der Dichte ist allerdings sehr gering, so daß eine Trennung dieser Blutzellen in einem isotonischen Gradienten nicht möglich ist.

Unter hypertonischen Bedingungen verlieren Lymphozyten schneller Wasser als Monozyten, resultierend in einer Zunahme ihrer Dichte. In einem hypertonischen Trennmedium ist es deshalb möglich, Monozyten aus Vollblut oder leukozytenreichem Plasma zu isolieren. Eine Isolierung humaner Blutmonozyten ist ebenfalls aus "Buffy coat" möglich. Hierbei wurden mononukleare Zellen unter isotonischen Bedingungen im Dichtegradienten getrennt und Monozyten anschließend in Gewebekulturflaschen (175 cm²/800 ml) durch ihre Adhärenz separiert.

Mit Hilfe der durchflußzytofluometrischen Analyse konnte nach Bestimmung von Größe und Granularität sowie nach spezifischer Antikörperfärbung eine Stimulation dieser Zellen durch Adhärenz nachgewiesen werden. Aufgrund dieser Stimulation wurden die Monozyten, die durch Adhärenz am Boden der Gewebekulturflaschen isoliert wurden, im folgenden als Monozyten/Makrophagen (stimulierte Monozyten) bezeichnet.

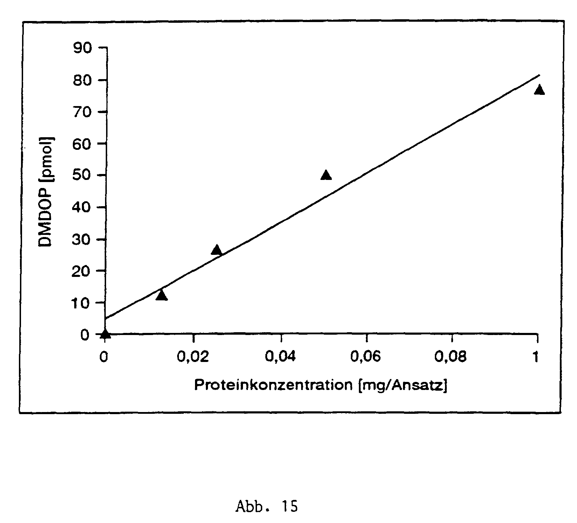

Bei enzymatischen Umsetzungen von AZT-DMDOPE durch die Membranfraktion humaner Monozyten, die im Verlauf ihrer Isolierung durch Adhärenz stimuliert wurden, konnte eine lineare Abhängigkeit bis zu einer Konzentration von 0.1 mg Protein/Ansatz festgestelit werden (Abb. 15). Die spezifische Aktivität betrug 8.8 ± 0.26 pmol mg⁻¹ min⁻¹ (n = 4) (Abb.16). Wurden die Monozyten direkt im hypertonischen Dichtegradienten isoliert, so zeigt sich bei der enzymatischen Spaltung lineares Verhalten nur bis zu einer Proteinkonzentration von 0.025 mg Protein/Ansatz. Bei höheren Proteinkonzentrationen blieb der Substratumsatz nahezu konstant. Die spezifische Aktivität wurde analog zu Umsetzungen mit Membranfraktionen nichtstimulierter humaner PBL aus dem linearen Bereich berechnet und betrug 3.2 + 0.60 pmol mg⁻¹ min⁻¹ (n = 4).

Zusammenfassend läßt sich feststellen, daß bei der Umsetzung von AZT-DMDOPE durch Enzyme der Membranfraktionen stimulierter humaner PBL und Monozyten höhere spezifische LCE-Aktivitäten beobachtet wurden als bei Umsetzung von AZT-DMDOPE durch Membranfraktionen nichtstimulierter Zellen (Abb. 16).

### d. Abhängigkeit der spezifischen LCE-Aktivität von der Inkubationszeit

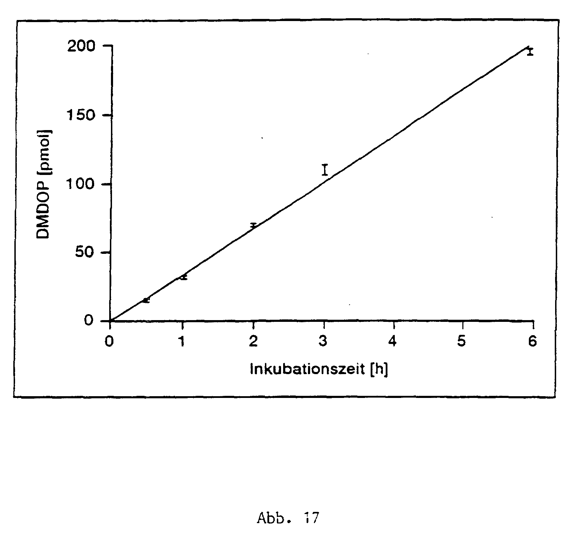

Die enzymatische Spaltung der Versuchssubstanz AZT-DMDOPE in Abhängigkeit von der Inkubationszeit wurde mit Membranfraktionen stimulierter humaner PBL als Enzymquelle durchgeführt. Die Reaktionsansätze wurden 0.5, 1, 2, 3, und 6 h bei 37 °C inkubiert und anschließend die Menge des Metaboliten DMDOP bestimmt. Nach graphischer Auftragung der Versuchsergebnisse konnte gezeigt werden, daß die Umsetzung von AZT-DMDOPE zu DMDOP über den gesamten Zeitraum der inkubation von 0.5 - 6 h Linearität zu erkennen ist (Abb. 17).

### e. Abhängigkeit der spezifischen LCE-Aktivität von zweiwertigen Metallkationen

Die Abhängigkeit der enzymatischen Spaitung von AZT-DMDOPE durch das LCE von zweiwertigen Metallkationen wurde mit Membranfraktionen stimulierter humaner PBL als Enzymquelle untersucht.

Die zweiwertigen Metallkationen wurden in einer Konzentration von 2 mM im LCE-Assay eingesetzt. Die Proteinkonzentration wurde mit 0.068 mg/Ansatz festgelegt.

Parallel wurde ein Ansatz mit EGTA mitgeführt. EGTA ist ein spezifischer Komplexbildner für Ca²⁺ das essentiell für die Aktivität der Phospholipase C ist. Nach einer Inkubationszeit von 2 h bei 37 °C wurde der Umsatz der Muttersubstanz gemessen und die spezifische Aktivität des LCE bestimmt (folgende Tabelle).

**Tabelle:**

| ***Spaltung von AZT-DMDOPE durch Membranfraktionen stimulierter humaner PBL in Abhängigkeit von EGTA und zweiwertigen Metall- kationen (Mittelwert* ± *SD, n* = 3 *Bestimmungen)*** | | | |
|---|---|---|---|
| **Effektor** **[2 mM]** | **DMDOP** **[pmol]** | **Spezifische Aktivität** **[pmol mg-1 min-1]** | **Inhibition** **[%]** |
| **EGTA** | **84.96 ± 11.18** | **10.41 ± 1.37** | **≡ 0** |
| **CaCl**_{**2**} | **50.50 ± 2.13** | **6.19 ± 0.26** | **41.0 ± 2.5** |
| **MgCl**_{**2**} | **84.36 ± 17.02** | **10.34 ± 2.08** | **0** |
| **ZnCl**_{**2**} | **0** | **0** | **100.0** |
| **MnCl**_{**2**} | **4.75 ± 0.63** | **0.58 ± 0.08** | **94** |

Die höchste spezifische Aktivität wurde bei Verwendung von EGTA und MgCl₂ mit 10.41 ± 1.37 (n = 3) bzw. 10.34 ± 2.08 pmol mg⁻¹ min⁻¹ (n = 3) erreicht.
Innerhalb der Meßgenauigkeit konnte also keine Inhibierung durch MgCl₂ nachgewiesen werden. Wurde im Enzym-Assay die CaCl₂ eingesetzt, so konnte eine spezifische Aktivität von 6.19 ± 0.26 pmol mg⁻¹ min⁻¹ (n = 3) bestimmt werden. Dies bedeutet im Vergleich zum Ansatz mit EGTA eine Hemmung der LCE-Aktivität von 41.0 ± 0.25 % (n = 3). Bei ZnCl₂ war innerhalb der Meßgenauigkeit kein DMDOP nachweisbar. ZnCl₂ und MnCl₂ führten zu einer totalen Hemmung der Umsetzung von AZT-DMDOPE zu DMDOP und AZT-MP.

### f. Bestimmung der apparenten Michaelis-Menten-Parameter K_{M} und vₘₐₓ

Zur Bestimmung der apparenten Michaelis-Menten-Parameter für das LCE wurde die enzymatische Spaltung von AZT-DMDOPE in Abhängigkeit steigender Substratkonzentrationen untersucht.

**Tabelle:**

| ***Apparente Michaelis-Menten-Parameter K***_{***M***} ***und v***_{***max***} ***des LCE in stimulierten und nichtstimulierten humanen PBL sowie humanen Monozyten und Monozyten*/*Makrophagen (stimulierte Monozyten)*** | | |
|---|---|---|
| | **v**_{**max**} **[pmol mg**^{**-1**} **min**^{**-1**}**]** | **KM** **[µM]** |
| **nichtstimulierte humane PBL** | **2.03 ± 0.19** | **5.51 ± 0.99** |
| **stimulierte humane PBL** | **15.29 ± 0.37** | **2.26 ± 0.16** |
| **humane Monozyten** | **1.61 ± 0.37** | **12.08 ± 4.13** |
| **humane Monozyten/Makrophagen** **(stimulierte Monozyten)** | **9.09 ± 0.44** | **4.63 ± 0.45** |

Hierfür wurden Membranfraktionen stimulierter und nichtstimulierter humaner PBL sowie humaner Monozyten und Monozyten/Makrophagen (stimulierte Monozyten) eingesetzt. Die Reaktionsansätze enthielten eine konstante Proteinmenge von 0.068 mg/Ansatz und wurden bei 37 °C für 2 h inkubiert. Anschließend wurde die Menge des Metaboliten DMDOP bestimmt.

### Beispiel 10:

### Versuchstiere

Zur Bestimmung der pharmakokinetischen Parameter der Versuchssubstanz AZT-DMDOPE wurden *In-vivo*-Experimente an weiblichen Balb/c-Mäusen (Charles River Wiga, Sulzfeld; Bormholtgard, Ry (Dänemark); Iffa Credo, L'Abresle (Frankreich)) durchgeführt. Die Tierlieferungen wurden vor Versuchsbeginn auf Virusantikörper (Mäuse Heptatisvirus, Reo-Virus, Paro-Virus) untersucht. In den Versuchen wurden nur Tiere eingesetzt, deren Antikörpertiter negativ war.

### Versuchstierhaltung

Die Tierhaltung erfolgte in vollklimatisierten Tierställen mit einer Raumtemperatur von 22 - 24 °C, einer relativen Luftfeuchtigkeit von 50 - 70 % und einem Tag-Nacht-Rhythmus von 12 Stunden. Durch Laminarflowboxen wurde im Tierstall 15 - 20 mal pro Stunde ein Luftaustausch gewährleistet. Den Tieren wurde eine Standarddiät (Ssniff, Soest) und über eine Flaschentränke Wasser *ad libitum* verabreicht.

### Beispiel 11:

### Zellkulturmethoden

### a. Kryokonservierung von Zellinien

Zur Kryokonservierung wurden Zellen in RPMI 1640 Medium auf eine Zelldichte von 5 x 10⁶ Zellen/ml eingestellt und 10 min bei 1600 rpm in der Minifuge T sedimentiert. Anschließend wurde das Zellsediment in einem gleichen Volumen an Kryokonservierungsmedium aufgenommen. Nach Resuspension des Zellsediments wurde jeweils 1 ml der Zellsuspension in Kryoröhrchen (1.8 ml) überführt und für 24 h bei -70 °C eingefroren. Zur endgültigen Lagerung wurden die Kryoröhrchen in einen Thermobehälter mit flüssigen N₂ überführt.

| | |
|---|---|
| Kryokonservierungsmedium | 60 % (v/v) RPMI 1640 Medium |
| | 0.05 mM 2-Mercaptoethanol |
| | 100 U/ml Penicillin |
| | 100 µg/ml Streptomycin |
| | 30 % (v/v) Fötales Kälberserum |
| | 10 % (v/v) DMSO |

### b. Stammkulturhaltung und Aufzuchtbedingungen

### Kultivierung von CEM-SS-Zellen

Die Kultivierung der Suspensionszellinie CEM-SS erfolgte in RPMI 1640 Komplettmedium I. Hierfür wurden 1 x 10⁷ Zellen in eine Gewebekulturflasche (83 cm²/260 ml) mit 50 ml Medium überführt. Anschließend wurde die Kultur für 3 Tage bei 37 °C und 5 % CO₂ inkubiert. Für die Zellpassage wurden die Zellen mit einer sterilen Pipette der Gewebekulturflasche entnommen und in der Minifuge T für 10 min bei 1600 rpm sedimentiert. Nach Resuspension der Zellen in Kultivierungsmedium und Bestimmung der Zellzahl durch Eosinfärbung in der Neubauer-Zählkammer wurden 1 x 10⁷ Zellen zur Passage in 50 ml frisches Medium eingesetzt.

### Kultivierung von P3X63Ag8.653-Zellen

Die Kultivierung von P3X63Ag8.653-Zellen erfolgte als Monolayer in RPMI 1640 Komplettmedium I. Hierfür wurden 5 x 10⁶ Zellen in eine Gewebekulturflasche (83 cm²/260 ml) mit 40 ml Medium überführt. Die Zellkultur wurde für 5 Tage bei 37 °C und 5 % CO₂ inkubiert bis der Boden der Gewebekulturflasche mit einem konfluenten Zellmonolayer bedeckt war. Für die Zellpassage wurden die adhärenten Zellen mit einem Zellschaber mechanisch vom Flaschenboden gelöst und anschließend durch Zentrifugation für 10 min bei 1600 rpm in der Minifuge T sedimentiert. Nach Resuspension der Zellen in Kultivierungsmedium wurde die Zellzahl durch Eosinfärbung in der Neubauer-Zählkammer bestimmt und 5 x 10⁶ Zellen zur Passage in 40 ml frisches Medium eingesetzt.

### c. Zellzahlbestimmungen

### Bestimmung der Zellzahl durch Eosinfärbung in der Neubauer-Zählkammer (Lindl und Bauer, 1989, Zell- und Gewebekultur, S. 75-90, Gustav Fischer Verlag, Stuttgart, New York)

Zur Bestimmung der Zellzahl in Suspensions- und Monolayerkulturen wurde in PBS eine Zellkonzentration von 0.1 - 1 x 10⁶ Zellen/ml eingestellt und eine Portion dieser Zellsuspension mit 20 µl Eosin versetzt. Anschließend wurde die Zellsuspension vorsichtig mit einer Pipette durchmischt, für 2 min bei Raumtemperatur inkubiert und in eine Neubauer-Zählkammer überführt.

Die Bestimmung der Zellzahl erfolgte unmittelbar nach der Inkubationszeit im Umkehr-Phasenkontrast-Mikroskop, wobei die Zellen innerhalb der vier großen Quadrate gezählt wurden. Vitale Zellen wurden hierbei im Gegensatz zu avitalen Zellen nicht angefärbt. Schwach angefärbte Zellen wurden als avital betrachtet.

Die Zahl vitaler Zellen/ml ergab sich aus der Anzahl nichtgefärbter Zellen der vier Großquadrate multipliziert mit dem Kammmerfaktor 10⁴ und dem Verdünnungsfaktor der Zellsuspension in der Eosinlösung.

### Bestimmung der Zellzahl durch elektronische Zählung im Coulter-Counter (Lindl und Bauer, 1989)

Die Messung der Zellzahl im Coulter-Counter beruht auf einem Durchfluß der Zellen zwischen zwei Platinelektroden. Passiert eine Zelle die Öffnung der beiden Elektroden, so ändert sich der Widerstand des durch die Elektroden fließenden Stroms proportional zur Größe der Zelle. Dies erzeugt einen Spannungsimpuls, der aufgezeichnet wird und somit eine Quantifizierung der Zellen erlaubt.

Zur Bestimmung der Zellzahl wurden 25 µl der Zellsuspension in 3 ml Iso-Osmol, einer Trägerflüssigkeit für den Coulter-Counter, überführt und in die Apparatur injiziert.

Bei dieser Art der Zellzählung wird die Gesamtzellzahl der Zellsuspension bestimmt. Eine Unterscheidung vitaler und avitaler Zellen ist nicht möglich.

### d. Ablösung adhärenter Zellen durch Trypsin/EDTA

Adhärente Zellen konnten auf enzymatischem Weg durch Behandlung mit Trypsin/EDTA vom Boden des jeweiligen Kulturgefäßes gelöst werden. Hierfür wurde der Kulturüberstand vorsichtig dekantiert und die Zellen zweimal mit vorgewärmten (37 °C) Kultivierungsmedium gewaschen. Zur Ablösung der Zellen vom Boden des Kulturgefäßes wurden diese für 5 min mit einer Trypsin/EDTA-Lösung (1 x) bei Raumtemperatur inkubiert. Das Volumen an Trypsin/EDTA-Lösung wurde hierbei so gewählt, daß der Boden des jeweiligen Kulturgefäßes 2 - 3 mm mit der enzymatischen Lösung bedeckt war. Nach Beendigung der Inkubationszeit wurden die Zellen durch vorsichtiges Schütteln der Flasche vom Boden gelöst, aus dem Kulturgefäß entfernt und mit einem gleichen Volumen an RPMI 1640 Komplettmedium I versetzt. Anschließend wurden die Zellen für 10 min bei 1600 rpm in der Minifuge T sedimentiert. Der Überstand wurde verworfen, das Zellsediment in kaltem PBS resuspendiert und unter gleichen Bedingungen erneut zentrifugiert. Die Zellen wurden dann entsprechend ihrer weiteren Verwendung in Kultivierungsmedium oder Puffer resuspendiert.

### e. Isolierung von mononuklearen Zellen aus Humanblut

### Isolierung von humanen PBL aus "Buffy coat" durch Zentrifugation im isotonischen Dichtegradienten (Böyum, 1968, Scand. J. Clin. Lab. Invest. 21 (Suppl. 97), 77-89; Böyum 1976, Scand. J. Clin. Lab. Invest. 5 (Suppl. 5), 5-15)

Zur Isolierung von humanen PBL wurde "Buffy coat" von gesunden Spendern gemischt und mit RPMI 1640 Komplettmedium II im Volumenverhältnis 1:2 verdünnt. In Polypropylenröhrchen (50 ml) wurden 20 ml kaltes Lymphozytentrennmedium vorgelegt und vorsichtig mit 15 ml des verdünnten "Buffy coats" überschichtet. Die Fraktionierung erfolgte durch Zentrifugation bei Raumtemperatur für 30 min und 1600 rpm (Beschleunigung 4/Bremse 4) in der Minifuge T. Die zwischen Probe und Trennmedium gut sichtbare Bande wurde mit Hilfe einer Combitip-Pipette gewonnen. Zur Entfernung des Trennmediums wurden die gesammelten Fraktionen bei Raumtemperatur für 10 min und 1600 rpm sedimentiert. Der Überstand wurde dekantiert und die Zellen entsprechend ihrer Verwendung dreimal mit RPMI 1640 Komplettmedium II oder kaltem PBS gewaschen.

### Stimulation und Kultivierung von humanen PBL

Nach Isolierung von humanen PBL mittels Zentrifugation im isotonischen Dichtegradienten war es möglich, periphere Lymphozytenkulturen anzulegen. Normalerweise sterben Blutzellen in Kultur relativ schnell ab. Lymphozyten können allerdings über mehrere Generationen in Kultur gehalten werden. Um eine Proliferation dieser Zellen zu erreichen, wurden sie mit dem Mitogen PHA-M, dem Lektinextrakt der roten Feuerbohne (*Phaseolus vulgaris*), stimuliert.

Zur Stimulation wurden die isolierten Zellen in Gewebekulturflaschen (175 cm²/800 ml) in RPMI 1640 Komplettmedium II auf eine Zelldichte von 1 x 10⁶ Zellen/ml eingestellt und für 72 h bei 37 °C und 5 % CO₂ inkubiert. Um eine zu große Zelldichte zu vermeiden, wurde nach 24 h die Zellsuspension mit Kultivierungsmedium im Volumenverhältnis 1:1 verdünnt. Nach Beendigung der Stimulationszeit wurde das Mitogen PHA-M durch dreimaliges Waschen mit RPMI 1640 Komplettmedium II entfernt. Zur Zellexpansion wurden die stimulierten Lymphozyten in RPMI 1640 Komplettmedium IV auf eine Zelldichte von 5 - 7 x 10⁶ Zellen/ml eingestellt und für 24 h kultiviert.

### Herstellung von leukozytenreichem Plasma aus Vollblut (Böyum, 1968)

Venöses Frischblut von gesunden Spendern wurde zur Verhinderung der Gerinnung im Volumenverhältnis 49:1 mit steriler 10 % (w/v) EDTA-Lösung versetzt. Um eine schnelle Vermischung dieser Komponenten zu erreichen, wurden dem Spender kleine Volumina von 8 - 9 ml Blut in Polystyrolröhrchen (14 ml) entnommen. Zur Herstellung von leukozytenreichem Plasma wurde EDTA-Blut und Dextran 75 im Volumenverhältnis 10:1 gemischt. Die beiden Komponenten wurden gut durchmischt, und die Erythrozyten bei Raumtemperatur über einen Zeitraum von 60 min sedimentiert. Anschließend wurde der fast klare Überstand, das leukozytenreiche Plasma, mit einer Pipette vorsichtig entnommen und zur Isolierung von Monozyten verwendet.

### Isolierung von humanen Monozyten aus leukozyten-reichem Plasma durch Zentrifugation im hypertonischen Dichtegradienten (Böyum, 1983, Scan. J. Clin. Lab. Invest. 17, 429-436)

In einer hypertonischen Lösung war es möglich, nichtstimulierte Monozyten aus Vollblut oder leukozytenreichem Plasma zu isolieren. Hierfür wurden in Polystyrolröhrchen (14 ml) 3 ml des hypertonischen Trennmediums NycoPrep 1.068 vorgelegt und vorsichtig mit 6 ml leukozytenreichem Plasma überschichtet. Die Röhrchen wurden verschlossen und in der Minifuge T bei Raumtemperatur für 15 min und 600 x g (Beschleunigung 4/Bremse 4) zentrifugiert. Nach der Zentrifugation wurde die klare Plasmaphase bis 5 mm über der breiten, diffusen Bande entfernt und verworfen. Die verbleibende Plasmaphase und die Hälfte der breiten, diffusen Bande wurde entnommen und die Zellen durch Zentrifugation in der Minifuge T für 7 min unter gleichen Bedingungen sedimentiert. Das Zellsediment wurde anschließend zur Entfernung von Resten des Trennmediums zweimal in 6 ml Waschlösung resuspendiert und gewaschen.

| | | |
|---|---|---|
| Nycoprep 1.068 | 13 % (w/v) | Nycodenz |
| | 0.58 % (w/v) | NaCl |
| | 5 (mM) | Tricine/NaOH pH 7.4 |
| | Dichte | 1.068 ± 0.001 g/ml (20 °C) |
| | Osmolarität | 335 ± 5 mOsm |

| | | |
|---|---|---|
| Waschlösung | 0.9 % (w/v) | NaCl |
| | 0.13 % (w/v) | EDTA |
| | 1 % (w/v) | BSA (Fraktion V) |

### Isolierung von humanen Monozyten aus "Buffy coat" durch Adhärenz (Andreesen et al., 1983, J. Immun. Methods 56, 295-304)

Aus "Buffy coat" wurden mononukleare Zellen durch Zentrifugation im isotonischen Dichtegradienten isoliert und dreimal mit serumfreiem RPMI 1640 Medium gewaschen. Zur Isolierung der Monozyten wurden die Zellen in Gewebekulturflaschen (175 cm²/800 ml) mit 20 ml RPMI 1640 Komplettmedium II auf eine Zelldichte von 5 x 10⁶ Zellen/ml eingestellt und für 45 min bei 37 °C und 5 % CO₂ inkubiert. Nichtadhärente Zellen wurden nach Beendigung der Inkubation durch Dekantieren des Mediums entfernt. Die Zellschicht am Boden der Gewebekulturflasche wurde anschließend zur Entfernung von Resten an Suspensionszellen zweimal mit temperiertem (37 °C), serumfreiem RPMI 1640 Medium gewaschen. Die Zellschicht wurde mit 30 ml RPMI 1640 Komplettmedium V versetzt und für weitere 24 h unter gleichen Bedingungen inkubiert. Die Ablösung der adhärenten Monozyten erfolgte durch eine Behandlung mit Trypsin/EDTA.

### Beispiel 12:

### Immunologische Methoden

### a. Durchflußzytofluometrische Analyse von Zellsubpopulationen aus Humanblut durch direkte und sequentielle Antikörperfärbung

Für die durchflußzytofluometrische Analyse wurden die zu charakterisierenden Zellen in FACS-PBS auf eine Zelldichte von 1 x 10⁷ Zellen/ml eingestellt. Die Antikörperstammlösungen wurden mit FACS-PBS im Volumenverhältnis 1:50 verdünnt. Für die direkte und sequentielle Antikörperfärbung wurden 50 µl der Zellsuspension (1 x 10⁷ Zellen/ml) in die Vertiefungen einer Mikrotiterplatte mit konischem Boden vorgelegt.

| Antikörper für die durchflußzytofluometrische Analyse mononuklearer humaner Blutzellen | | | |
|---|---|---|---|
| **Antikörper** | **Spezifität** | **Fluoreszenzfarbstoff** | **Antikörperfärbung** |
| **Anti-Leu-12** **(CD19)** | **Leu-12 (CD19)** **B-Lymphozyten** | **PE** | **direkt** |
| **Anti-Leu-5b** **(CD2)** | **Leu-5 (CD2)** **T-Lymphozyten** | **FITC** | **direkt** |
| **Anti-Monozyt** **(CD14)** | **gp 55** **Monozyten** **Makrophagen** | **-** | **sequentiell** |
| **Anti-Leu-2a** **(CD8)** | **Zytotoxische-T-Lymphozyten** | **FITC** | **direkt** |
| **Anti-Leu-3a** **(CD4)** | **Helfer-T-Lymphozyten** | **PE** | **direkt** |

Anschließend wurde die Zellsuspension mit einem gleichen Volumen des Antikörpers versetzt, der im Falle einer direkten Antikörperfärbung mit einem fluoreszierenden Farbstoff gekoppelt ist. Die Ansätze wurden für 30 min bei 4 °C inkubiert und anschließend zur Sedimentation der Zellen bei 1400 rpm und Raumtemperatur für 2 min in der Minifuge T zentrifugiert.

Die Überstände wurden entfernt, und das Zellsediment zur Entfernung von Resten des nichtgebundenen Antikörpers in 200 µl FACS-PBS resuspendiert und erneut, wie beschrieben, zentrifugiert. Der Überstand wurde entfernt und das Zellsediment im Falle einer direkten Antikörperfärbung in 100 µl FACS-PBS resuspendiert.
Bei einer sequentiellen Antikörperfärbung wurde nach Beendigung der Zentrifugation das Zellsediment in 100 µl einer Lösung des mit Phycoerythrin markierten Antikörpers Ziege-Anti-Maus Ig-R-PE (3 µg/ml) aufgenommen und für 30 min bei 4 °C inkubiert. Reste des nichtgebundenen Antikörpers wurden, wie beschrieben, durch Waschen mit 200 µl FACS-PBS entfernt und das Zellsediment zur durchflußzytofluometrischen Zellanalyse in 100 µl FACS-PBS resuspendiert.

### b. Bestimmung der Zellvitalität durch Propidiumiodidfärbung im durchflußzytofluometrischen Analysator

Die Bestimmung der Anzahl vitaler und avitaler Zellen durch durchflußzytofluometrische Analyse ist nach Färbung der Zellen mit dem fluoreszierenden Farbstoff Propidiumjodid möglich. Dieser Farbstoff wird nur von avitalen Zellen aufgenommen. Hierfür wurde die Zellsuspension in PBS auf eine Konzentration von 0.5 - 1 x 10⁶ Zellen/ml eingestellt. Aus dieser Zellsuspension wurden 180 µl entnommen und mit 20 µl einer Propidiumjodidlösung (5 µg/ml) versetzt. Nach einer Inkubationszeit von 5 min wurden 100 µl der Lösung in den Zellanalysator injiziert. Durch eine statistische Analyse im Lysis II-Programm des Zellanalysators konnte die prozentuale Verteilung der vitalen und avitalen Zellen bestimmmt werden.

### c. Quantitative Bestimmung von AZT in Zellextrakten mittels ¹²⁵J-AZT-Radioimmuno-Assay

Zur quantitativen Bestimmung von AZT wurde ein kommerzieller ¹²⁵J-AZT RIA Test-Kit verwendet. Puffer und Lösungen wurden gemäß den Angaben des Herstellers angesetzt und im Test-Kit eingesetzt. Die Reaktionsansätze wurden nach untenstehendem Schema (Tab. ) pipettiert, vorsichtig gemischt und für 2 h bei Raumtemperatur inkubiert. Anschließend wurden zur Präzipitation des Komplexes aus ¹²⁵J-AZT und des AZT-Antikörpers (aus Kaninchen) 500 µl des Ziege-Anti-Kaninchen-Antikörpers hinzugefügt. Die Ansätze wurden gemischt und erneut bei Raumtemperatur für 30 min inkubiert.

| Pipettierschema des ¹²⁵J-AZT-Radioimmuno-Assays zur quantitativen Bestimmung von AZT in Zellextrakten | | | | |
|---|---|---|---|---|
| | **nicht bindender Standard** | **Nullstandard** | **Standard** | **Probe** |
| **Nullstandard** **[µl]** | **300** | **200** | **-** | **-** |
| **Standard** **[µl]** | **-** | **-** | **200** | **-** |
| **Probe** **[µl]** | **-** | **-** | **-** | **200** |
| ^{**125**}**J-AZT** **[µl]** | **100** | **100** | **100** | **100** |
| **AZT-Antikörper** **[µl]** | **-** | **100** | **100** | **100** |

Zur Sedimentation des Präzipitationskomplexes wurden die Ansätze anschließend bei 1000 x g für 20 min zentrifugiert. Nach Dekantieren des Überstandes wurde die Radioaktivität des Sedimentes für 60 sec im Flüssigkeits-Szintillations-Analysator gemessen. Zur Ermittlung des Absolutwertes wurde die Radioaktivität von 100 µl ¹²⁵J-AZT nach einer Inkubation von 2 h bei Raumtemperatur unter gleichen Bedingung bestimmt. Die Konzentrationen an AZT in der biologischen Matrix wurde mit Hilfe einer Eichkurve ermittelt, die mit Standards definierter Substanzkonzentrationen des Test-Kits erstellt wurde.

### Beispiel 13:

### Charakterisierung der intrazellulären enzymatischen Spaltung von BM 21.1290 Na

Die Ergebnisse der In-vitro-Pharmakokinetikstudien mit AZT-DMDOPE bzw. AZT in stimulierten und nichtstimulierten humanen PBL sowie thymidinkinasedefizienten P3X63Ag8.653-Zellen deuten auf eine intrazelluläre enzymatische Spaltung von AZT-DMDOPE unter direkter Freisetzung von AZT-MP und des korrespondierenden Thioetherlipidteiles DMDOP hin. Diese intrazelluläre enzymatische Spaltung sollte in weiteren Experimenten auf subzellulärer Ebene durch einen direkten Nachweis der entsprechenden Metabolite belegt werden. Für eine eindeutige Charakterisierung der Spaltung war somit die Identifizierung der bisher bekannten Metabolite notwendig, die aus der Muttersubstanz AZT-DMDOPE entstehen können. Hierzu gehören die Substanzen DMDOP, AZT und AZT-MP, sowie die Substanzen , die durch Oxidation des Schwefels im Thioetherlipidteil aus der Muttersubstanz AZT-DMDOPE entstehen.

### a. Entwicklung von Methoden zur Bestimmung der R_{f}-Werte von AZT-DMDOPE und dessen potentieller Metabolite

Die Identifizierung der potentiellen Metabolite von AZT-DMDOPE war mit Hilfe der DC möglich. Hierfür wurden die nicht radioaktiv markierten Reinsubstanzen mit Hilfe verschiedener Trennsysteme analysiert und anschließend eine Bestimmung der R_{f}-Werte durchgeführt.

Die Substanzen wurden hierfür in einem Gemisch aus Essigsäureethylester und Methanol (1:1, v/v) mit einer Konzentration von 4 mg/ml gelöst. Mit Hilfe einer Mikrokapillare wurden 5 µl dieser Lösungen auf die stationäre Phase aufgetragen und analysiert.

Thioetherlipide wie AZT-DMDOPE , DMDOP, sowie solche, die durch Oxidation des Schwefels entstehen, konnten mit Hilfe eines jodhaltigen Reagenz markiert werden, das den Thioetherlipidteil dieser Verbindungen anfärbt. AZT und AZT-MP konnten aufgrund ihrer Absorption im ultravioletten Bereich bei 254 nm ausschließlich auf DC-Platten mit Fluoreszenzindikator sichtbar gemacht werden. AZT-DMDOPE , die Substanz mit Thioetherlipidteil und chromophorer Gruppe, waren beiden Arten der Detektion zugänglich.

Für die dünnschichtchromatographische Analyse der Substanzen wurden drei Trennsysteme etabliert, die sich hinsichtlich ihrer stationären und mobilen Phasen unterscheiden.

Im ersten Trennsystem, dem IBA-System, mit Kieselgel 60 als stationärer Phase, konnte die Muttersubstanz AZT-DMDOPE neben DMDOP identifiziert werden. Als mobile Phase wurde ein Gemisch aus 2-Propanolin-Butylacetat:Aqua bidest. (10:6:4, v/v/v) eingesetzt.

Eine Weiterentwicklung dieses Trennsystems wurde durch die Verwendung von 2-Propanol:n-Butylacetat:Aqua bidest.:Eisessig (3:5:1:1, v/v/v/v) als mobile Phase (IBAE-System) erreicht. Die Substanzen AZT-DMDOPE und DMDOP konnten hierbei, wie im IBA-Trennsystem beschrieben, auf Kieselgel 60 DC-Platten getrennt werden. Zusätzlich konnten AZT und AZT-MP im UV-Licht bei 254 nm und Verwendung einer stationären Phase aus Kieselgel 60 mit Fluoreszenzindikator getrennt werden. Aufgrund des AZT-Teils war ebenfalls eine Detektion der Muttersubstanz AZT-DMDOPE sowie deren Oxidationsprodukte möglich.

Nach Visualisierung der Substanzen auf der DC-Platte wurden die R_{f}-Werte bestimmt.

Zur Unterscheidung der Substanz AZT-DMDOPE von DMDOP stand ein weiteres Trennsystem zur Verfügung. Hierbei wurde als mobile Phase ein Gemisch aus n-Heptan und Essigsäureethylester (4:1, v/v) und als stationäre Phase Kieselgel 60 verwendet. Die Detektion wurde mit dem bereits beschriebenen jodhaltigen Reagenz durch Anfärben des Thioetherlipidteils der Substanzen durchgeführt.

Im Vergleich zum IBA- und IBAE-System konnte für die Substanz DMDOP mit diesem Trennsystem ein deutlich niedriger R_{f}-Wert bestimmt werden. Grund hierfür ist eine im Vergleich zum IBA- und IBAE-System unpolare mobile Phase, bestehend aus n-Heptan und Essigsäureethylester. Die Nachweisgrenze der Substanzen nach dünnschichtchromatographischer Analyse und Detektion mit einem jodhaltigen Reagenz wurde in allen Trennsystemen mit 0.1 µg/ml bestimmt.

Zusammenfassend kann festgehalten werden, daß mit Hilfe der beschriebenen dünnschichtchromatographischen Trennsysteme alle Substanzen, die nach derzeitigem Kenntnisstand als potentielle Metabolite von AZT-DMDOPE in Frage kommen, sich über ihre R_{f}-Werte eindeutig charakterisieren lassen.

**Tab.**

| ***R***_{***f***}***-Werte von AZT-DMDOPE und DMDOP nach Trennung im IBA- System (2-Propanol:n-Butylacetat:Aqua bidest., 10:6:4, v*/*v*/*v) und Kieselgel 60 als stationärer Phase (Mittelwerte ± SD, n =* 6 *Bestimmungen)*** | |
|---|---|
| **Testsubstanz** | **R**_{**f**}**-Wert** |
| **AZT-DMDOPE** | **0.68 ± 5.2 x 10**^{**-3**} |
| **DMDOP** | **0.93 ± 0.00** |

**Tab.**

| ***R***_{***f***}***-Werte von AZT-DMDOPE, DMDOP, AZT und AZT-MP nach Trennung im IBAE System (2-Propanol:n-Butylacetaf:Aqua bidest:Eis- essig,* 3:5:1:1, *v*/*v*/*v*/*v) und Kieselgel 60 bzw. 60 F 254 als stationärer Phase (Mittelwerte ± SD, n = 6 Bestimmungen)*** | |
|---|---|
| **Testsubstanz** | **R**_{**f**}**-Wert** |
| **AZT-DMDOPE** | **0.57 ± 1 x 10**^{**-2**} |
| **DMDOP** | **0.99 ± 0.00** |
| **AZT** | **0.85 ±0.00** |
| **AZT-MP** | **0.14 ± 0.00** |

### b. Enzymatische Spaltung von AZT-DMDOPE durch Zellhomogenate von stimulierten und nichtstimulierten humanen PBL sowie von CEM-SS-Zellen

Die Spaltung von AZT-DMDOPE wurde mit Hilfe eines Enzym-Assays mit [¹⁴C]-AZT-DMDOPE und AZT-DMDOPE als Substrat untersucht.
Als Enzymquelle wurden zunächst Zellhomogenate von CEM-SS-Zellen und humanen PBL verwendet.

**Tab.**

| **R**_{**f**}**-Werte von AZT-DMDOPE und DMDOP nach dünnschichtchromatographischer Trennung im HE-System (n-Heptan:Essigsäureethylester, 4:1, v/v) und Kieselgel 60 als stationärer Phase (Mittelwerte ± SD, n = 6 Bestimmungen)** | |
|---|---|
| **Testsubstanz** | **R**_{**f**}**-Wert** |
| **AZT-DMDOPE** | **0.0 ± 0.00** |
| **DMDOP** | **0.36 ± 0.00** |

Letztere wurden sowohl nach Stimulation mit PHA-M als auch in nichtstimuliertem Zustand direkt nach ihrer Isolierung homogenisiert und im Enzym-Assay eingesetzt.

Zur Herstellung der Zellhomogenate wurden Zellsuspensionen mit einer Dichte von 5 x10⁷ Zellen/ml 50 mM Tris (pH 7.4) im Glashomogenisator mechanisch aufgeschlossen. Der Zellaufschluß wurde hierbei im Umkehr-Phasenkontrast-Mikroskop optisch kontrolliert.

**Tab.**

| ***R***_{***f***}***-Werte der Substanzen 1 - 4 (Abb. 18) nach 1, 3, 6 und 24 h Inkubation und enzymatischer Spaltung von [***^{***14***}***C]-AZT-DMOPE durch Zellhomogenate von 5 x 10***^{***7***} ***stimulierten und nicht- stimulierten humanen PBL und CEM-SS-Zellen. Trennung im IBA*/*IBAE-System mit Kieselgel* 60 *als stationärer Phase (Mittelwerte ± SD, n = 4 Bestimmungen)*** | | | | |
|---|---|---|---|---|
| | **R**_{**f**}**-Wert** **Substanz 1** | **R**_{**f**}**-Wert** **Substanz 2** | **R**_{**f**}**-Wert** **Substanz 3** | **R**_{**f**}**-Wert** **Substanz 4** |
| **CEM-SS** | **0.41 ± 2.5 x 10**^{**-2**} | **0.56 ± 2.5 x 10**^{**-2**} | **0.86 ± 5.8 x 10**^{**-3**} | **0.96 ± 0.0** |
| **stimulierte humane PBL** | **-** | **0.65 ± 9.5 x 10**^{**-3**} | **0.86 ± 5.8 x 10**^{**-3**} | **0.94 ± 0.0** |
| **nichtstimulierte humane PBL** | **-** | **0.65 ± 1.7 x 10**^{**-2**} | **-** | **0.92 ± 1.2 x 10**^{**-2**} |

Zunächst wurde 1 ml dieses Zellhomogenales in dem Enzym-Assay ohne vorherige Bestimmung der Proteinkonzentration eingesetzt. Als Substrat wurden 0.98 nmol [¹⁴C]-AZT-DMDOPE (1.67 kBq) eingesetzt.

Zur Gewährleistung einer Substratsättigung wurden weitere 50 nmol der nichtradioaktiv markierten Verbindung hinzugefügt. [¹⁴C]-AZT-DMDOPE trägt die radioaktive Markierung im Thioetherlipidteil und erlaubt somit eine eindeutige Identifikation der potentiellen Metabolits [¹⁴-C]DMDOP.

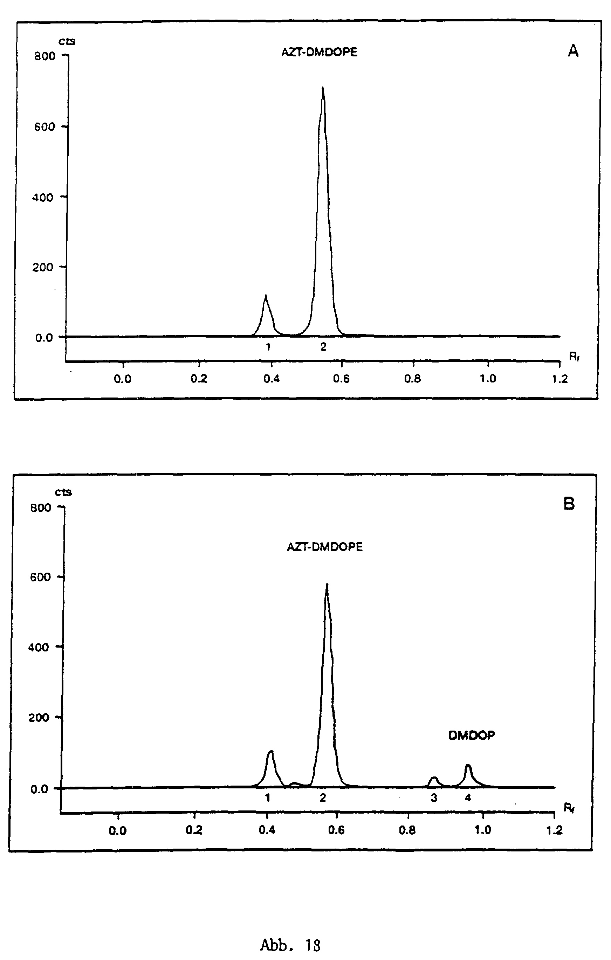

Die Ansätze wurden für 1, 3, 6 und 24 h im Wasserbad bei 37 °C inkubiert. Als Vergleich wurde ein Ansatz ohne Zugabe von Zellhomogenat mitgeführt. Anschließend wurden die Spaltprodukte mit Diethylether:2-Propanol (9:1, v/v) extrahiert und dünnschichtchromatographisch im IBA- und IBAE-System analysiert (Abb. 18). Nach Trennung der Substanzen wurde zur Detektion der radioaktiv markierten Substanzen die DC-Platte 15 min im Radio-DC-Analysator gemessen. Eine eindeutige Identifizierung der Metaboliten war schließlich durch Bestimmung der R_{f}-Werte möglich.

**Tab.**

| ***R***_{***f***}***-Werte der Substanzen 1 und 2 (Abb. 18) nach 1, 3, 6 und 24 h Inkubation von [***^{***14***}***C]-BM 21.1290 Na. Trennung im IBA*/*IBAE- System mit Kieselgel* 60 *als stationärer Phase (Mittelwerte ± SD*, *n = 4 Bestimmungen)*** | | |
|---|---|---|
| | **R**_{**f**}**-Wert** **Substanz 1** | **R**_{**f**}**-Wert** **Substanz 2** |
| **CEM-SS** | **0.41 ± 5.7 x 10**^{**-3**} | **0.56 ± 5.8 x 10**^{**-3**} |
| **stimulierte humane PBL** | **-** | **0.65 ± 9.6 x 10**^{**-3**} |
| **nichtstimulierte humane PBL** | **-** | **0.65 ± 1.9 x 10**^{**-2**} |

Die dünnschichtchromatographische Analyse der enzymatischen Spaltprodukte von AZT-DMDOPE durch Zellhomogenate stimulierter und nichtstimulierter humaner PBL wurde durch Anwendung des IBA-Trennsystems durchgeführt. In den sich anschließenden Experimenten mit CEM-SS-Zellhomogenat wurde die Analyse der Spaltprodukte im IBAE-Trennsystem durchgeführt. Da von beiden Trennsystemen die R_{f}-Werte der Reinsubstanzen ermittelt wurden, war ein direkter Vergleich der Ergebnisse zur Identifikation der Substanzen nach enzymatischer Spaltung von AZT-DMDOPE möglich.

Nach enzymatischer Spaltung von [¹⁴C]-AZT-DMDOPE durch Zellhomogenate stimulierter bzw. nichtstimulierter humaner PBL wurden drei unbekannte Substanzen im DC-Chromatogramm detektiert. Mit R_{f}-Werten von 0.65 ± 9.5 x 10⁻² (n = 4) und 0.65 ± 1.7 x 10⁻² (n = 4) für stimulierte und nichtstimulierte humane PBL konnte Substanzpeak 2 eindeutig der Muttersubstanz [¹⁴C]-AZT-DMDOPE zugeordnet werden. Die R_{f}-Werte von Substanzpeak 4 mit 0.94 ± 0.00 (n = 4) und 0.92 ± 1.2 x 10⁻² (n = 4) waren identisch mit den Werten von DMDOP, die durch DC-Analyse der Reinsubstanzen bestimmt wurden. Substanzpeak 3 mit einem R_{f}-Wert von 0.86 ± 5.8 x 10⁻³ (n = 4) konnte keiner der bekannten Substanzen zugeordnet werden. Die Ergebnisse wurden durch Analyse der enzymatischen Spaltung von [¹⁴C]-AZT-DMDOPE durch CEM-SS-Zellhomogenate bestätigt. Substanzpeak 2 und 4 mit R_{f}-Werten von 0.56 ± 2.5 x 10⁻² (n = 4) und 0.96 ± 0.00 (n = 4) konnten eindeutig als AZT-DMDOPE bzw. DMDOP identifiziert werden, während Substanzpeak 1 mit einem R_{f}-Wert von 0.41 ± 2.5 x 10⁻² (n = 4) einem Oxidationsprodukt zugeordnet werden konnte. Für Substanzpeak 3 konnte auch im IBAE-System nach Vergleich der R_{f}-Werte keine Zuordnung getroffen werden (Abb. 18). Weiterhin konnte gezeigt werden, daß nach Inkubation von [¹⁴C]-AZT-DMDOPE ohne Zugabe von Zellhomogenat nur die Muttersubstanz zu detektieren war (Abb. 18).

Aus der dünnschichtchromatographischen Analyse der Reaktionsansätze konnte somit der Beweis geführt werden, daß die Substanz DMDOP aus der Muttersubstanz freigesetzt wird.

### Beispiel 14:

### 9-(β-D-Arabinofuranosyl)-2-fluoradenin-5'-phosphorsäure-(3-dodecylmercapto-2-decyloxy)-propylester (Fludarabin-Konjugat)

34.8 g (0.07 mol) Phosphorsäure-(3-dodecylmercapto-2-decyloxy)-propylester wurden in 130 ml abs. Pyridin gelöst, unter Stickstoff mit 15 g Methansulfonsäurechlorid versetzt und 3 Stunden bei Raumtemperatur gerührt. Dann wurden 20 g Fludarabin vorsichtig eingetragen und die Lösung weitere 48 Stunden bei Raumtemperatur gerührt. Fludarabin wurde analog zu J.Heterocyclic Chem. 16, 157 (1979) hergestellt.
Nach Hydrolyse des Reaktionsgemisches durch Zugabe von 30 ml 1 M Triethylammoniumbicarbonat-Lösung und einstündiges Rühren wurde das Pyridin im Vakuum entfernt, der Rückstand zwischen 200 ml t-Butylmethylether (MTB) und 150 ml Wasser verteilt, die organische Phase abgetrennt und im Rotationsverdampfer eingedampt.
Der Rückstand wurde chromatographisch an RP-18 mit Methanol/0.02M Acetatpuffer pH 4 8/2 als Eluens gereinigt.
Die produktenthaltenden Fraktionen wurden bis auf den Wasseranteil eingeengt, mit MTB extrahiert und die MTB-Phase mit Natriummethylat-Lösung gegen Friscolyt auf pH 7 eingestellt.
Nach Abdampfen des Lösungsmittels wurde der Rückstand in Aceton suspendiert, der amorphe Niederschlag abgesaugt und getrocknet.
Ausbeute: 23.7 g (43 %). Amorph. Rf = 0.45 (DC-Laufmittel: Isopropanol/Butylacetat/ Wasser/Ammoniak 50/30/15/5).

### Beispiel 15:

### 2-Chlor-2'-desoxyadenosin-5'-phosphorsäure-(3-dodecylmercapto-2-decyloxy)-propylester (Cladribin-Konjugat)

Cladribin-Konjugat wurde analog zu Beispiel 14 unter Verwendung von 4.2 g Phosphorsäure-(3-dodecylmercapto-2-decyloxy)-propylester, 3 g Methansulfonsäure, 100 ml Pyridin und 2 g Cladribin mit 35 % Ausbeute hergestellt. Rf = 0.41 (Laufmittel wie in Bsp. 14). Cladribin wurde analog zu J.Am.Chem.Soc. 106, 6379 (1984) hergestellt.

### Beispiel 16:

### [3-(2-Desoxy-β-D-erythro-pentofuranosyl)-3,6,7,8-tetrahydroimidazo[4,5-d][1,3]diazepin-8-ol]-5'-phosphorsäure-(3-dodecylmercapto-2-decyloxy)-propylester (Pentostatin-Konjugat)

Pentostatin-Konjugat wurde analog zu Beispiel 14 unter Verwendung von 4.2 g Phosphorsäure-(3-dodecylmercapto-2-decyloxy)-propylester, 3 g Methansulfonsäure, 100 ml Pyridin und 2.1 g Pentostatin mit 27 % Ausbeute hergestellt. Rf = 0.52 (Laufmittel wie in Bsp. 14). Pentostatin wurde analog zu J.Org.Chem. 47, 3457 (1982) bzw. J.Am.Chem.Soc. 101, 6127 (1979) hergestellt.

## Patentansprüche

1. Lipid-Cleavage-Enzymkomplex (LCE) frei von Phospholipase C-Aktivität, **dadurch gekennzeichnet, dass** er das Konjugat AZT-DMDOPE [(3'-Desoxy-3'-azidothymidin)-5'-phosphorsäure-(3-dodecylmercapto-2-decyloxy)-propylester] in AZT-MP [3'-Desoxy-3'-azidothymidinmonophosphat] und DMDOP [(3'-Dodecylmercapto-2-decyloxy)-propanol] spaltet oder das Konjugat FLT-DMDOPE [(3'-Desoxy-3'-fluorthymidin)-5'-phosphorsäure-(3-dodecylmercapto-2-decyloxy)-propylester] in FLT-MP [3'-Desoxy-3'fluorthymidin)-5'-monophosphat] und DMDOP spaltet oder das Konjugat 5-FU-DMDOPE [5-Fluoruridin-5'-phosphorsäure-(3-dodecylmercapto-2-decyloxy)-propylester in 5-FU-MP [5-Fluoruridinmonophosphat] und DMDOP spaltet, wobei LCE **durch folgende Merkmale gekennzeichnet ist**
- membranständig, isolierbar aus humanen peripheren Blutleukozyten
- Molekulargewicht 120.000-160.000, bestimmt nach der SDS-PAGE-Methode
- aktivierbar durch die Substanz D609 (Tricyclodecan-9-yl-xanthogenat)
- inhibierbar durch Ca²⁺, Zn²⁺ und Mn²⁺.

2. LCE nach Anspruch 1, **dadurch gekennzeichnet, dass** er aus Leukozyten, Monozyten, Tumorzellen, Nierenzellen, Lymphozyten, Zellen des Immuno/lymphatischen Systems oder Makrophagen erhältlich ist.

3. LCE nach einem der Ansprüche 1 oder 2, wobei der LCE bei Konjugaten vom Typ L-B-D, in denen L ein lipidähnlicher Rest, B eine Phosphatbrücke oder eine Thiophosphatbrücke und D eine pharmakologisch aktive Substanz bedeutet oder B-D ein Wirkstoffphosphonat darstellt, eine Spaltung der kovalenten Bindung zwischen dem Lipidteil L und dem Rest -B-D induziert.

4. LCE nach einem der Ansprüche 1, 2 oder 3, **dadurch gekennzeichnet, dass** die Aktivität des LCE bei der Spaltung des Konjugates in aktivierten Immunzelten, humanen Leukozyten, Monozyten, Tumorzellen, Lymphozyten, Nierenzellen, Zellen des Immuno/lymphatischen Systems oder Makrophagen mindestens um den Faktor 2 höher ist im Vergleich zu nicht aktivierten Zellen.

5. Isolierte sirbzelluläre Systeme zur Durchführung von In-vitro-Untersuchungsverfahren, **dadurch gekennzeichnet, dass** die subzellulären Systeme mit dem LCE nach einem der Ansprüche 1 oder 2 angereichert sind.

6. Verfahren zum Auffinden oder Identifizieren von Verbindungen, die als Substrat oder Ligand des LCE nach einem der Ansprüche 1 oder 2 fungieren, **dadurch gekennzeichnet, dass** folgende Schritte getrennt oder zusammen umfasst werden,
a) der LCE wird bereitgestellt
b) der LCE wird mit der Verbindung inkubiert
c) Spaltprodukte werden mit einem geeigneten System nachgewiesen.

7. Verfahren zum Auffinden oder Identifizieren von Verbindungen, die als Aktivatoren oder Inhibitoren des LCE nach Anspruch 1 oder 2 fungieren, **dadurch gekennzeichnet, dass** folgende Schritte getrennt oder zusammen umfasst werden,
a) der LCE wird bereitgestellt
b) der LCE wird mit dem Stoff inkubiert
c) ein Substrat oder Ligand wird zugegeben
d) eine Änderung der Enzymaktivität wird mit einem geeigneten System nachgewiesen.

8. Verwendung des LCE nach einem der Ansprüche 1 oder 2 zum Auffinden oder zur Identifizierung von Substraten, Liganden, Inhibitoren oder Aktivatoren des LCE.

9. Verwendung von subzellulären Systemen nach Anspruch 5 zum Auffinden oder zur Identifizierung von Substraten, Liganden, Inhibitoren oder Aktivatoren des LCE.

10. Diagnostisches Mittel enthaltend LCE nach einem der Ansprüche 1, 2, 3 oder 4 zur Durchführung von Bestimmungsverfahren für Substrate, Liganden, Aktivatoren oder Inhibitoren des lipidspaltenden LCE

11. Mittel zum Auffinden von Substanzen die als Substrate oder Liganden des LCE fungieren, enthaltend eine ausreichende Menge des isolierten oder in Zellpräparaten angereicherten LCE nach einem der Ansprüche 1, 2, 3 oder 4, sowie geeignete Stabilisierungsmittel.

12. Verwendung eines kovalenten Konjugates eines Lipid-Derivates und einer pharmakologisch aktiven Substanz der Formel L-B-D zur Herstellung eines Arzneimittels zur gezielten Freisetzung des Restes -B-D in geeigneten Zielzellen, ausgewählt aus der Gruppe der folgenden Substanzen:
2-Fluor-9-(b-D-arabinofuranosyl)adenin-5'-phosphorsäure-(3-dodecylmercapto-2-decyloxypropyl)ester,
2-Chlor-2 -desoxyadenosin-5'-phosphorsäure-(3-dodecylmercapto-2-decyloxypropyl)ester,
3-(2-Desoxy-b-D-erythropentofuranosyl)-3,6,7,8-tetrahydro-imidazo-[4,5-d][1,3]-diazepin-8-ol-5'-phosphorsäure-(3-dodecylmercapto-2-decyloxypropyl)ester.

13. Lipidkonjugate der Formel I vom Typ L-B-D ausgewählt aus der Gruppe der folgenden Substanzen:
2-Fluor-9-(b-D-arabinofuranosyl)adenin-5'-phosphorsäure-(3-dodecylmercapto-2-decyloxypropyl)ester,
2-Chlor-2 -desoxyadenosin-5'-phosphorsäure-(3-dodecylmercapto-2-decyloxypropyl)ester,
3-(2-Desoxy-b-D-erythropentofuranosyl)-3,6,7,8-tetrahydro-imidazo-[4,5-d][1,3]-diazepin-8-ol-5'-phosphorsäure-(3-dodecylmercapto-2-decyloxypropyl )ester.

14. Arzneimittel enthaltend ein Lipidkonjugat vom Typ L-B-D ausgewählt aus der Gruppe der folgenden Substanzen:
2-Fluor-9-(b-D-arabinofuranosyl)adenin-5'-phosphorsäure-(3-dodecylmercapto-2-decyloxypropyl)ester,
2-Chlor-2 -desoxyadenosin-5'-phosphorsäure-(3-dodecylmercapto-2-decyloxypropyl)ester,
3-(2-Desoxy-b-D-erythropentofuranosyl)-3,6,7,8-tetrahydro-imidazo-[4,5-d][1,3]-diazepin-8-ol-5'-phosphorsäure-(3-dodecylmercapto-2-decyloxypropyl)ester
als Wirkstoff sowie weitere pharmazeutische Hilfs- oder Trägerstoffe.

## Claims

1. Lipid cleavage enzyme complex (LCE) free of phospholipase C activity, **characterized in that** it cleaves the conjugate AZT-DMDOPE [(3'-deoxy-3'-azidothymidine)- 5'-phosphoric acid-(3-dodecyl-mercapto-2-decyloxy)-propyl ester] into AZT-MP [3'-deoxy-3'-azido-thymidine-monophosphate] and DMDOP [(3-dodecyl-mercapto-2-decyloxy)-propanol] or the conjugate FLT-DMDOPE [(3'-deoxy-3'-fluorothymidine)-5'-phosphoric acid-(3-dodecylmercapto-2-decyloxy)-propyl ester] into FLT-MP [3'-deoxy-3'-fluoro-thymidine-5'-monophosphate] and DMDOP or the conjugate 5-FU-DMDOPE [5-fluorouridine-5'-phosphoric acid-(3-dodecylmercapto-2-decyfoxy)-propyl ester] into 5-FU-MP [5-fluorouridine monophosphate] and DMDOP, whereby LCE is **characterized by** the following properties:
- membranous, can be isolated from human peripheral blood leucocytes
- molecular weight 120.000-160.000 determined by the SDS-PAGE method
- can be activated by the substance D609 (Tricyclodecan-9-yl-xanthogenat)
- can be inhibited by Ca²⁺, Zn²⁺ and MN²⁺.

2. LCE as claimed in claim 1, wherein it is obtainable from leucocytes, monocytes, tumour cells, kidney cells, lymphocytes, cells of the immuno/lymphatic system or macrophages.

3. LCE as claimed in one of the claims 1 or 2, wherein in the case of conjugates of the type L-B-D in which L represents a lipid-like residue, B represents a phosphate bridge or a thiophosphate bridge and D denotes a pharmacologically active substance or B-D represents an active substance phosphonate the LCE induces a cleavage of the covalent bond between the lipid moiety L and the residue -B-D.

4. LCE as claimed in one of the claims 1, 2, or 3, wherein the activity of the LCE during cleavage is at least 2-fold higher in activated immune cells, human leucocytes, monocytes, tumour cells, lymphocytes, kidney cells, cells of the immuno/lymphatic system or macrophages compared to non-activated cells.

5. Isolated subcellular systems for carrying out in vitro test procedures wherein the subcellular systems are enriched with the LCE as claimed in one of the claims 1 or 2.

6. Method to find or identify compounds which act as a substrate or ligand of the LCE as claimed in one of the claims 1 or 2, wherein it comprises the following separate or simultaneous steps
a) the LCE is prepared
b) the LCE is incubated with the compound
c) cleavage products are detected using a suitable system.

7. Method to find or identify compounds which act as activators or inhibitors of the LCE as claimed in claim 1 or 2, wherein it comprises the following separate or simultaneous steps
a) the LCE is prepared
b) the LCE is incubated with the substance
c) a substrate or ligand is added
d) a change in the enzyme activity is detected in a suitable system.

8. Use of the LCE as claimed in one of the claims 1 or 2 to find or to identify substrates, ligands, inhibitors or activators of the LCE.

9. Use of subcellular systems as claimed in claim 5 to find or to identify substrates, ligands, inhibitors or activators of the LCE.

10. Diagnostic agent containing LCE as claimed in one of the claims 1, 2, 3 or 4 for carrying out methods of determination for substrates, ligands, activators or inhihibitors of the lipid cleaving LCE.

11. Agent for finding substances which act as a substrate or ligand of the LCE, containing an adequate amount of the LCE in an isolated form or concentrated in cell preparations as claimed in one of the claims 1, 2, 3 or 4 as well as suitable stabilizers.

12. Use of a covalent conjugate of a lipid derivative and a pharmacologically active substance of the formula L-B-D to produce a pharmaceutical agent for the targeted release of the residue -B-D in suitable target cells whereby the conjugate is selected from the group comprising
2-fluoro-9-(b-D-arabinofuranosyl)adenine-5'-phosphoric acid-(3-dodecylmercapto-2-decyloxypropyl) ester,
2-chloro-2'-deoxyadenosine-5'-phosphoric acid-(3-dodecylmercapto-2-decyloxypropyl) ester,
3-(2-deoxy-b-D-erythropentofuranosyl)-3,6,7,8-tetrahydro-imidazo-[4,5-d][1,3]-diazepin-8-ol-5'-phosphoric acid-(3-dodecylmercapto-2-decyloxy-propyl) ester.

13. Lipid conjugate of formula I of type L-B-D selected from the group of the following substances:
2-fluoro-9-(b-D-arabinofuranosyl)adenine-5'-phosphoric acid-(3-dodecylmercapto-2-decyloxypropyl) ester,
2-chloro-2'-deoxyadenosine-5'-phosphoric acid-(3-dodecylmercapto-2-decyloxypropyl) ester,
3-(2-deoxy-b-D-erythropentofuranosyl)-3,6,7,8-tetrahydro-imidazo-[4,5-d][1,3]-diazepin-8-ol-5'-phosphoric acid-(3-dodecylmercapto-2-decyloxy-propyl) ester.

14. Pharmaceutical agent comprising a lipid conjugate of the type L-B-D selected from the group of the following substances:
2-fluoro-9-(b-D-arabinofuranosyl)adenine-5'-phosphoric acid-(3-dodecylmercapto-2-decyloxypropyl) ester,
2-chloro-2'-deoxyadenosine-5'-phosphoric acid-(3-dodecylmercapto-2-decyloxypropyl) ester,
3-(2-deoxy-b-D-erythropentofuranosyl)-3,6,7,8-tetrahydro-imidazo-[4,5-d][1,3]-diazepin-8-ol-5'-phosphoric acid-(3-dodecylmercapto-2-decyloxy-propyl) ester.
as active ingredient as well as further pharmaceutical auxiliary or carrier substances.

## Revendications

1. Complexe d'enzyme de clivage de lipides (LCE) exempt d'activité de phospholipase C, **caractérisé en ce qu'**il dissocie le système conjugué AZT-DMDOPE [ester (3-dodécylmercapto-2-décyloxy)-propylique de l'acide (3'-désoxy-3'-azidothymidine)-5'-phosphorique] en AZT-MP [3'-désoxy-3'-azidothymidinemonophosphate] et en DMDOP [(3'-dodécylmercapto-2-décyloxy)-propanol] ou le système conjugué FLT-DMDOPE [ester (3-dodécylmercapto-2-décyloxy)propylique de l'acide (3'-désoxy-3'-fluorothymidine)-5'-phosphorique] en FLT-MP [3'-désoxy-3'-fluorothymidine)-5'-monophosphate] et en DMDOP ou le système conjugué 5-FU-DMDOPE [ester (3-dodécylmercapto-2-décyloxy)propylique de l'acide 5-fluorouridine-5'-phosphorique en 5-FU-MP [5-fluorouridinemonophosphate] et en DMDOP, le LCE étant **caractérisé par** les caractéristiques suivantes
- positionné dans la membrane, isolable à partir de leucocytes humains du sang périphérique
- poids moléculaire 120000 à 160000, déterminé selon le procédé SDS-PAGE
- activable par la substance D609 (Tricyclodécan-9-yl-xanthogénate)
- pouvant être inhibé par Ca²⁺, Zn²⁺ et Mn²⁺.

2. LCE selon la revendication 1, **caractérisé en ce qu'**il peut être obtenu à partir de leucocytes, de monocytes, de cellules tumorales, de cellules rénales, de lymphocytes, de cellules du système immunologique/lymphatique ou de macrophages.

3. LCE selon l'une quelconque des revendications 1 ou 2, le LCE induisant une dissociation de la liaison covalente entre la partie lipide L et le résidu -B-D dans les conjugués de type L-B-D, dans lesquels L signifie un résidu analogue aux lipides, B signifie un pont phosphate ou thiophosphate et D une substance pharmacologiquement active ou B-D représente un phosphonate de substance active.

4. LCE selon l'une quelconque des revendications 1, 2 ou 3, **caractérisé en ce que** lors de la dissociation du système conjugué dans des cellules immunes activées, des leucocytes activés, des monocytes activés, des cellules tumorales activées, des lymphocytes activés, des cellules rénales activées, des cellules activées du système immunologique/lymphatique ou des macrophages activés, l'activité du LCE est supérieure d'au moins un facteur 2 par rapport aux cellules non activées.

5. Systèmes sous-cellulaires isolés destinés à la réalisation de procédés d'analyse in vitro, **caractérisés en ce que** les systèmes sous-cellulaires sont enrichis en LCE selon l'une quelconque des revendications 1 ou 2.

6. Procédé pour mettre en évidence ou identifier des composés qui fonctionnent comme substrat ou ligand du LCE selon l'une quelconque des revendications 1 ou 2, **caractérisé en ce que** les étapes suivantes sont comprises séparément ou ensemble
a) mise à disposition du LCE
b) incubation du LCE avec le composé
c) mise en évidence des produits de dissociation avec un système approprié.

7. Procédé pour mettre en évidence ou identifier des composés qui fonctionnent comme activateurs ou inhibiteurs du LCE selon la revendication 1 ou 2, **caractérisé en ce que** les étapes suivantes sont comprises séparément ou ensemble
a) mise à disposition du LCE
b) incubation du LCE avec la substance
c) addition d'un substrat ou d'un ligand
d) mise en évidence d'une modification de l'activité enzymatique avec un système approprié.

8. Utilisation du LCE selon l'une quelconque des revendications 1 ou 2 pour mettre en évidence ou identifier des substrats, des ligands, des inhibiteurs ou des activateurs du LCE.

9. Utilisation de systèmes sous-cellulaires selon la revendication 5 pour mettre en évidence ou identifier des substrats, des ligands, des inhibiteurs ou des activateurs du LCE.

10. Agent diagnostique contenant le LCE selon l'une quelconque des revendications 1, 2, 3 ou 4 pour réaliser des procédés de détermination de substrats, de ligands, d'activateurs ou d'inhibiteurs du LCE dissociant les lipides.

11. Agent pour mettre en évidence des substances qui agissent comme substrats ou ligands du LCE, contenant une quantité suffisante du LCE selon l'une quelconque des revendications 1, 2, 3 ou 4, isolé ou enrichi dans des préparations cellulaires a insi que d es a gents d e stabilisation appropriés.

12. Utilisation d'un système conjugué covalent d'un dérivé lipidique et d'une substance pharmacologiquement active de formule L-B-D pour la préparation d'un médicament destiné à la libération ciblée du résidu -B-D dans des cellules cibles appropriées, choisi dans le groupe des substances suivantes :
ester 3-dodécylmercapto-2-décyloxypropylique de l'acide 2-fluoro-9-(b-D-arabinofurannosyl)adénine-5'-phosphorique,
ester 3-dodécylmercapto-2-décyloxypropylique de l'acide 2-chloro-2-désoxyadénosine-5'-phosphorique,
ester 3-dodécylmercapto-2-décyloxypropylique de l'acide 3-(2-désoxy-b-D-érythropentofurannosyl)-3,6,7,8-tétrahydro-imidazo-[4,5-d][1,3]-diazépin-8-ol-5'-phosphorique.

13. Système conjugué lipidique de formule I du type L-B-D choisi dans le groupe des substances suivantes :
ester 3-dodécylmercapto-2-décyloxypropylique de l'acide 2-fluoro-9-(b-D-arabinofurannosyl)adénine-5'-phosphorique,
ester 3-dodécylmercapto-2-décyloxypropylique de l'acide 2-chloro-2-désoxyadénosine-5'-phosphorique,
ester 3-dodécylmercapto-2-décyloxypropylique de l'acide 3-(2-désoxy-b-D-érythropentofurannosyl)-3,6,7,8-tétrahydro-imidazo-[4,5-d][1,3]-diazépin-8-ol-5'-phosphorique.

14. Médicament contenant un système conjugué lipidique du type L-B-D choisi dans le groupe des substances suivantes :
ester 3-dodécylmercapto-2-décyloxypropylique de l'acide 2-fluoro-9-(b-D-arabinofurannosyl)adénine-5'-phosphorique,
ester 3-dodécylmercapto-2-décyloxypropylique de l'acide 2-chloro-2-désoxyadénosine-5'-phosphorique,
ester 3-dodécylmercapto-2-décyloxypropylique de l'acide 3-(2-désoxy-b-D-érythropentofurannosyl)-3,6,7,8-tétrahydro-imidazo-[4,5-d][1,3]-diazépin-8-ol-5'-phosphorique
comme substance active ainsi que d'autres adjuvants ou substances support pharmaceutiques.
